# EUROPEAN PATENT SPECIFICATION

(11) **EP 2 475 242 B1**
(45) Date of publication and mention of the grant of the patent: **22.03.2017**
(21) Application number: 10755208.5
(22) Date of filing: 12.08.2010
(51) Int. Cl.: A01H 4/00, C12N 15/82, A01H 5/12, A01H 5/00

(54) **METHOD FOR PRODUCING LEAFY BIOMASS IN CULTURE**
KULTURMETHODE ZUR HERSTELLUNG VON BLATTARTIGER BIOMASSE
PROCÉDÉ DE PRODUCTION DE BIOMASSE FOLIAIRE EN CULTURE

(30) Priority: 11.09.2009 US 241613 P
(43) Date of publication of application: 18.07.2012
(73) Proprietor: Imperial Innovations Limited, London SW7 2PG (GB)
(72) Inventor: MICHOUX, Franck, 45200 Amilly (FR); NIXON, Peter, London SE24 9NA (GB); MCCARTHY, James, Gerard, 37210 Noizay (FR)
(74) Representative: Potter Clarkson LLP
(86) International application number: PCT/GB2010/001537
(87) International publication number: WO 2011/030083

(56) References cited:
- MICHOUX F ET AL: "Contained and high-level production of recombinant protein in plant chloroplasts" NEW BIOTECHNOLOGY, vol. 25, 1 September 2009 (2009-09-01), page S294, XP026461679 ELSEVIER BV, NL ISSN: 1871-6784 DOI: 10.1016/J.NBT.2009.06.668 [retrieved on 2009-08-12]
- ESPINOSA P ET AL: "Production of pineapple transgenic plants assisted by temporary immersion bioreactors" PLANT CELL REPORTS, vol. 21, no. 2, 1 August 2002 (2002-08-01), pages 136-140, XP002463447 SPRINGER VERLAG, DE ISSN: 0721-7714 DOI: 10.1007/S00299-002-0481-9
- HANHINEVA KATI J ET AL: "Production of transgenic strawberries by temporary immersion bioreactor system and verification by TAIL-PCR" BMC BIOTECHNOLOGY, vol. 7, no. 1, 19 February 2007 (2007-02-19), page 11, XP021023591 BIOMED CENTRAL LTD. LONDON, GB ISSN: 1472-6750 DOI: 10.1186/1472-6750-7-11 cited in the application
- HANHINEVA K ET AL: "Shoot regeneration from leaf explants of five strawberry (Fragaria x ananassa) cultivars in temporary immersion bioreactor system" IN VITRO CELLULAR & DEVELOPMENT BIOLOGY. PLANT, GAITHERSBURG, MD, US, vol. 41, no. 6, 1 November 2005 (2005-11-01), pages 826-831, XP002463446 ISSN: 1054-5476 DOI: 10.1079/IVP2005714
- ETIENNE H ET AL: "Temporary immersion systems in plant micropropagation" PLANT CELL, TISSUE AND ORGAN CULTURE, vol. 69, no. 3, 1 June 2002 (2002-06-01), pages 215-231, XP002463450 SPRINGER, NL ISSN: 0167-6857 DOI: 10.1023/A:1015668610465 cited in the application
- ETIENNE-BARRY D ET AL: "Direct sowing of Coffea arabica somatic embryos mass-produced in a bioreactor and regeneration of plants" PLANT CELL REPORTS, vol. 19, no. 2, December 1999 (1999-12), pages 111-117, XP002609598 ISSN: 0721-7714
- GATICA-ARIAS ANDRES M ET AL: "Plant regeneration via indirect somatic embryogenesis and optimisation of genetic transformation in Coffea arabica L. cvs. Caturra and Catuai" ELECTRONIC JOURNAL OF BIOTECHNOLOGY, vol. 11, no. 1, January 2008 (2008-01), pages 1-12, XP002609599 ISSN: 0717-3458

## Description

The present invention relates to a method for producing leafy biomass in culture.

The listing or discussion of an apparently prior-published document in this specification should not necessarily be taken as an acknowledgment that the document is part of the state of the art or is common general knowledge.

The production of biomass in culture is useful for the production of genetically engineered polypeptides, for the production of endogenous plant products, including medicinal products, polysaccharides, lignins and lipids, for the production of novel simple and complex chemicals not naturally found in plants through metabolite engineering, including new forms of polysaccharides, lignins, sugars, aromatic and aliphatic compounds, and for capturing carbon dioxide. The biomass may also be used for fuel in some circumstances.

WO 00/57690 relates to the micropropagation and production of phytopharmaceutical plants from differentiated plant pieces. In particular WO 00/57690 relates to the stimulation of small pieces of differentiated cells taken from an adult plant to produce new plantlets which can be grown to fully-formed phytopharmaceutical-producing plants capable of growth normal plant growth in typical plant growth media (e.g. soil, compost).

WO 01/94602 relates to a method for regenerating plants and uses thereof to multiply and/or transform plants using solid growth media. The plants resulting from the methods described in WO 01/94602 are viable plants that may grow under normal growth in typical plant growth media (e.g. soil and compost).

WO 2008/028115 relates to high-throughput methods for producing large numbers of transgenic corn plants in a short space of time by the use of a single container system for transgenesis, and growth into a viable plant. The corn plants produced are viable plants with root, stem and leaf structures and that are capable of normal plant growth in typical plant growth media (e.g. soil, compost).

Gatica-Arias et al 2008 (Electron. J. Biotechnol., 11(1)) relates to a protocol for *Coffea arabica* L. cvs. Caturra and Catuai plant regeneration via indirect somatic embryogenesis (ISE). That document also relates to a biolistic mediated genetic transformation protocol for Catuai callus aggregates.

The use of temporary liquid immersion culture systems (e.g. temporary immersion bioreactors or TIBs) is known, for example from Etienne & Berthouly (2002) Plant Cell, Tissue and Organ Culture 69, 215-231, Hanhineva & Kärenlampi (2007) BMC Biotechnology 7, 11-23, and also from Ducos et al (2007) In Vitro Cellular & Developmental Biology - Plant 43: 652-659. For example, Hanhineva & Kärenlampi (2007) describes the use of a TIB for production of transgenic strawberry plants, wherein the resulting plants comprise an exogenous gene and comprise both root and shoot formation, such that they would be capable of normal plant growth e.g. in soil or compost.

One of the main reasons why researchers have chosen plants for expressing biopharmaceuticals and other high-value proteins is the formidable scale-up possibility and very low maintenance costs that are associated with plant growth. However, the use of transgenic plants has its drawbacks, with public concern about the transfer of transgenes to surrounding non-transgenic crops and the possibility of food chain contamination (Fox, 2003).

For a long time, it was assumed that the plastid genome in most species was absent in pollen and was inherited maternally (Hagemann, 2004; Zhang *et al,* 2003; Scott and Wilkinson, 1999). Consequently, insertion of genes into the chloroplast genome, or plastome, to generate transplastomic plants, was considered to provide an intrinsic natural barrier to the pollen-mediated flow of transgenes. However, several recent publications have shown that the leak in chloroplast DNA containment is more frequent and widespread than originally thought. For example, transfer of chloroplast DNA to the pollen was estimated to reach 0.03% in *Setaria italica* (foxtail) (Wang *et al,* 2004), 0.01 to 0.00029% in tobacco (Ruf *et al*, 2007; Svab and Maliga, 2007) and 0.0039% in *Arabidopsis thaliana* (Azhagiri and Maliga, 2007).

Another concern is the possibility of chloroplast DNA being transferred to the nuclear genome over time (Sheppard *et al,* 2008), from where it could be passed on to a nearby non-transgenic species, in the same way as for a classic nuclear transformant. A frequency of one chloroplast DNA transfer to the nuclear DNA in every 16,000 pollen grains was detected in tobacco (Huang *et al,* 2003). Taking into account the fact that between 5,000 to 16,000 tobacco plants can be grown per acre, depending on the tobacco species, the risk of chloroplast DNA transfer to the nucleus is not negligible.

Concerns have also been raised that antibiotic-resistance cassettes, such as the *aadA* gene, which is used to select for chloroplast transformants, could be transferred to soil bacteria (Monier *et al,* 2007) and bacteria found in the gut of feeding insects (Brinkmann and Tebbe, 2007).

To circumvent any environmental issues that could result from planting transplastomic seeds in the field, one solution is to produce recombinant proteins in plant cell suspension cultures grown under contained conditions. Indeed, plant cell suspensions have been modified to express a large number of heterologous proteins (reviewed in Hellwig *et al,* 2004). Plant cell suspensions display several advantages over whole plants for the production of recombinant proteins, such as a shorter period of time before harvest, fully controlled growth and independence from weather conditions or diseases. Current good manufacturing practices, cGMP, based on bacterial production systems, can also be applied easily, leading to a quicker regulatory approval by the Federal Drug Administration (FDA) or by the European Agency for the Evaluation of Medicinal Products (EMEA) (reviewed in Ma *et al,* 2003; Fischer *et al,* 2004; Twyman *et al,* 2003).

Like bacteria, plant cell suspension cultures are inexpensive to grow and maintain. They are also intrinsically safe, because they neither harbour human pathogens nor produce endotoxins. Plant cell suspensions can be maintained in simple, synthetic media, but can synthesize complex multimeric proteins just like animal cells. In contrast to field-grown plants, the performance of cultured plant cells is independent of the climate, soil quality, season and day length. There is no risk of contamination with mycotoxins, herbicides or pesticides (Doran, 2000) and there are fewer by-products (e.g. fibres, oils, waxes, phenolic compounds). Perhap s the most important advantage of plant cell suspension cultures over whole plants is the much simpler procedures for product isolation and purification (Fischer *et al,* 1999).

However, the main disadvantages of plant cell suspension cultures are the slow growth and the usually low yields of recombinant protein produced by nuclear transformation (Hellwig *et al,* 2004). Another weakness is the fact that the productivity of plant cell cultures can vary considerably, with recombinant protein levels usually ranging from 0.0064% to 4% of total soluble protein (TSP), although in exceptional cases up to 20% of TSP can be achieved (Huang *et al,* 2001).

In general, chloroplast transformation better yields of recombinant protein than classic nuclear transformation. For example, the B-subunit of *E. coli* heat-labile enterotoxin (LTB) was expressed through both nuclear and plastid transformation in tobacco. The resulting yield was 250 times higher when the enterotoxin gene was inserted in the plastid genome (Kang *et al*, 2003). Similarly, when the cholera toxin B antigen (CTB) was expressed from nuclear and plastid DNA, antigen production in the tobacco chloroplast was 410 times higher than from the nucleus (Daniell *et al,* 2001). Even if chloroplast transformation seems to be superior for the over-expression of some proteins, only one report has been published on the possible production of recombinant GFP in transplastomic higher plant cell suspensions (Langbecker *et al,* 2004). This study described the plastid transformation of dark-grown tobacco plant cell cultures, but no estimation of expression potential was performed.

In the work described in the Examples, the expression levels of a plastid-encoded recombinant protein have been investigated, in this case a variant of the Green Fluorescent Protein GFP+ (Scholz *et al,* 2000) in leaf tissue, callus and cell suspensions grown under various conditions. The results indicate that expression in cell suspension cultures is a feasible route for high-level and contained expression of a foreign protein in the chloroplast although levels of expression are much less than that in plant leaves. There is also described the development a new expression system, based on temporary immersion bioreactors, which is able to produce extremely high-levels of recombinant protein starting from cell suspension cultures, and able to produce of high levels of leafy biomass from undifferentiated plant cells.

The invention is defined by the claims. A first aspect of the invention provides a method for producing leafy biomass from undifferentiated plant cells, the method comprising providing undifferentiated plant cells, contacting them with an agent that promotes differentiation of the cells into leafy tissue and growing the cells in a temporary liquid immersion culture system.

By "undifferentiated plant cells" we include the meaning that the cells show substantially no signs of being differentiated into any particular plant tissue such as shoot or leaf, and that they will remain in that state for at least one month under conditions where no agent which induces differentiation of undifferentiated cells is present, in particular there should be no agent that induces differentiation of undifferentiated cells into shoots. The undifferentiated cells may be transgenic or non-transgenic.

Typically, the undifferentiated cells can be derived from a permanent callus or callus material. A permanent callus is a cell culture of undifferentiated plant cells. Such permanent callus cells remain in an undifferentiated form for at least one month.

Undifferentiated cells can also be derived *in-vitro* from differentiated plant material, such as leaves, stems, flowers, seeds or roots, which are cut and placed in contact with certain plant hormones, such as Auxins.

When this plant material has been in contact with the hormones, calli will form in some areas of the plant material. The calli that are induced by hormones on differentiated plant material are not considered to be a permanent callus.

The step of providing the undifferentiated cells where the plant is not a transgenic plant comprises:
placing cut pieces of a plant (plant material) in contact with plant hormones, the plant hormones then generate calli at the edge of those cut pieces;
the callus is then subcultured as a callus and maintained without any selection.

The cut plant material may be all or part of a root, leaf, stem, flower or seed.

The step of providing the undifferentiated cells where the plant is a transplastomic plant comprises:
introducing the transgenic nucleic acid molecule into a chloroplast of a plant cell by a method of homologous recombination targeted to chloroplast DNA.
inducing the plant cell containing the transgenic nucleic acid molecule to form a callus of undifferentiated cells; and
propagating the callus under conditions effective to achieve homoplastomy.

The callus of the current invention work is a permanent callus, having been cultivated and maintained for at least one month as undifferentiated cells

Preferably, the only cells that are present when contacting with the agent are undifferentiated cells. Typically, at least 90%, or 95%, or 99%, or 99.9% or 99.99% of the cells present when contacting with the agent are undifferentiated cells.

Preferably, substantially all leafy and leaf like biomass material is produced upon differentiation of the undifferentiated cells following contact with the agent. Typically, the plant material produced upon treatment of the undifferentiated cells with the agent should be at least 50% leafy biomass, preferably 70%, and more preferably greater than 85%. By "leafy" and "leaf like" biomass" we include the meaning that the plant material is in the form of leaf or "leaf like" tissue. These leafy tissues are distinguished from other plant tissue by the shape of the tissue pieces, the number of chloroplasts and the significant photosynthetic activity. For example, for any given plant, leaf material has a higher number of chloroplasts and developing chloroplasts, as counted by confocal microscopy analysis of the plant tissue, and these chloroplasts have higher photosynthetic activity (determination of Fv/Fm with fluorometer) and higher chlorophyll content (by analysis of extracted pigments by absorption spectrophotometry) than chloroplasts in non-leaf material, as detected by the absorption of carbon dioxide by the plant tissue. Such methods of determination are well known to the skilled person as for example as described in (Baker (2008) Ann. Rev. Plant Biol. 59: 89-113).

The temporary liquid immersion culture system may be any such system as are known in the art (for example see Etienne & Berthouly (2002) Plant Cell, Tissue and Organ Culture 69, 215-231, Hanhineva & Kärenlampi (2007) BMC Biotechnology 7, 11-23, and also from Ducos et al (2007) In Vitro Cellular & Developmental Biology - Plant 43: 652-659. Typically, the systems contain a porous solid substrate upon which the cells reside (e.g. a net or a sponge or foam) which is immersed in liquid growth medium for short periods of time as discussed further below.

The plant cells may be cells from a monocotyledon or a dicotyledon.

Suitable dicotyledon plants include any of a tobacco, potato, tomato, bean, soybean, carrot, cassava, or *Arabidopsis.*

Suitable monocotyledon plants include any of corn, rye, oat, millet, sugar cane, sorghum, maize, wheat or rice.

In a preferred embodiment, the plant cells are from a medicinal plant in which the main medicinal product is produced in the leaves. It will be appreciated that the method represents an advantageous approach to obtaining such medicinal products by extracting them from the leafy biomass.

Suitable medicinal plants include any of *Atropa sp, Hyoscyamus sp, Datura sp, Papaver sp, Scopolia sp, Digitalis sp, Macuna sp, Taxus sp, Camptotheca sp, Cephalotaxus sp,* or *Catharanthus sp. Artemisia* sp, such as *Artemisia annua.* Medicines that may be derived from such medicinal plants include, but are not limited to Tropane Alkaloids, such
as atropine, scopolamine, and hyoscyamine and their precursors and derivatives; Morphinan Alkaloids, such as codeine, morphine, thebaine, norsanguinarine, sanguinarine, and cryptopine and their precursors and derivatives; Cardenolides such as digoxigenin, digitoxigenin, gitoxigenin, Diginatigenin, Gitaloxigenin and their precursors and derivatives; L-DOPA (L-3,4-dihydroxyphenylalanine) and its precursor and derivatives; Antitumor compounds such as taxol and its precursor and derivatives, Camptothecin and its derivatives, homoharringtonine, harringtonine, isoharringtonine and cephalotaxin and their precursors and derivatives; and Vinca Alkaloids such as vinblastine, vincristine, vindoline, catharanthine, their precursors and derivatives; malaria drugs, such as Artemisinin, its precursors and derivatives.

The medicinal compounds produced by the leafy biomass may be incorporated into pharmaceutical compositions by combination with pharmaceutically acceptable excipients, diluents or carriers.

In a further preferred embodiment, the plant may be an energy crop. By energy plants, we mean plant species used in the production of biofuels including ethanol or biodiesel. The current invention allows for a continuous production of biomass that can be employed for a continuous production of biofuel, independent from the season and plant species. The biomass generated can endogenously contain relatively elevated levels of polysaccharides, for use in fermentation based ethanol production processes, or relatively high levels of one or more lipids that can be further processed for the production of biodiesel. These elevated levels of advantageous compounds can also be generated in the biomass by genetic engineering. Suitably, the plant is any of *Miscanthus sp, Jatropha sp, Panicum sp,* Willow, palm tree, maize, cassava, or Poplar.

The agent that promotes differentiation of the cells into leafy tissue is typically a plant hormone (phytohormone or plant growth substance), and preferably a cytokinin. Cytokinins are a group of chemicals that primarily influence cell division and shoot formation but also have roles in delaying cell senescence, are responsible for mediating auxin transport throughout the plant, and affect internodal length and leaf growth. Auxins are compounds that positively influence cell enlargement, bud formation and root initiation. They also promote the production of other hormones and in conjunction with cytokinins, they control the growth of stems, roots, fruits and convert stems into flowers.

The cytokinin may be any natural or artificial cytokinin belonging to the adenine-type or the phenylurea-type. Preferably, the cytokinin is any of adenine, kinetin, zeatin, 6-benzylaminopurine, diphenylurea, thidiazuron (TDZ) and their respective derivatives which have cytokinin activity

The agents may promote, induce, and provoke differentiation such that shoots grow rapidly, preferably in an exponential manner, from any single undifferentiated plant cells derived from callus/cell suspension of the invention. Such shoots develop into leafy or leaf like biomass.

Preferably the agent that promotes differentiation of the cells into leafy tissue is thidiazuron (TDZ).

Conveniently, the agent may be used in combination with another plant hormone, such as an auxin, such as the naturally occurring auxins, 4-chloro-indoleacetic acid, phenylacetic acid (PAA), indole-3-butyric acid and indole-3-acetic acid; or the synthetic auxin analogues 1-naphthaleneacetic acid (NAA), 2,4-dichlorophenoxyacetic acid.

Typically, the agent is added in the culture medium at a concentration of from 0.01 to 100µM. Preferably the concentration is between 0.1 and 10 µM.

The agent may be added at the start of or during the temporary liquid immersion culture step.

Any suitable immersion regime may be selected, for example to optimise the production of leafy biomass or to optimise the concentration of a particular product in the leafy biomass, such as a polypeptide or medicinal product of interest. Typically, the immersion time varies from 1 to 30 minutes every 2 to 24 hours of culture. Preferably, the immersion time is between 1 and 10 minutes every 2 to 6 hours.

The skilled person will readily be able to select the most appropriate immersion culture parameters such as time, temperature and growth media based on the plant species and origin in order to generate a specific biomass for a specific purpose in the most effective manner i.e. at the most appropriate speed, quantity and quality.

The volume of liquid in the temporary liquid immersion culture may be any convenient volume but typically is from 1 to 10,000 litres. Alternatively, the volume may be between 1 and 5,000 litres, 1 and 1,000 litres, or 1 and 500 litres.

The vessel containing the temporary liquid immersion culture system may be any convenient size, and typically is from 1 to 10,000 litres. Alternatively, the volume may be between 1 and 5,000 litres, 1 and 1,000 litres, or 1 and 500 litres.

In one embodiment of the invention, the plant cells are not genetically engineered. As is well know, plants produce endogenously many important products in their leaves such as medicinal products as described above, as well as oils, pigments, antioxidants, simple and complex biochemicals such as sugars (carbohydrates), lipids, amino acids, volatile aromatic compounds, and flavours/flavour precursors.

The plant material of interest may also be capable of concentrating, capturing, or degrading, toxic pollutants in a sample, such as in a feed water source (plant based *in-vitro* decontamination/purification).

The plant material may also be used to transform one compound contained in the temporary reaction solution into one or more other compounds.

In another embodiment of the invention, the plant cells are genetically engineered, for example to express a polypeptide. The polypeptide may be any polypeptide of interest, but preferably is any one of a therapeutic polypeptide, an enzyme, a growth factor, an immunoglobulin, a hormone, a structural protein, a protein involved in stress responses of a plant, a biopharmaceutical, a peptide, or a vaccine antigen. When the polypeptide is an enzyme it may be used to alter the metabolism of the leafy material, thereby allowing the generation of novel polymers and metabolites. One or more polypeptides may also be expressed inside the leafy material to amplify the ability of the leafy tissue to purify or degrade pollutants found in a sample, such as a water source.

The genetically engineered plant cell (recombinant or transgenic plant cell) may be (i) a nuclear transformed plant cell in which the exogenous nucleic acid (transgene) resides in the nucleus; (ii) a transplastomic plant cell in which the exogenous nucleic acid (transgene) resides in a plastid, such as a chloroplast; or (iil) a plant cell that is both nuclear transformed and transplastomic.

Methods of making nuclear transformed plants and transplastomic plants are well known in the art. For example, nucleic acid molecules may be introduced into plant cells using particle bombardment, micro-injection, PEG-electroporation, agrobacterium mediated transformation, plant viruses and so on (see e.g. Birch 1997, Maliga 2004, Gleba et al., 2008)

It is preferred if the plant is a transplastomic plant.

Plants may be transformed in a number of art-recognised ways. Those skilled in the art will appreciate that the choice of method might depend on the type of plant targeted for transformation. Examples of suitable methods of transforming plant cells include microinjection (Crossway et al., BioTechniques 4:320-334 (1986)), electroporation (Riggs et al., Proc. Natl. Acad. Sci. USA 83:5602-5606 (1986), *Agrobacterium-mediated* transformation (Hinchee et al, Biotechnology 6:915-921 (1988);), direct gene transfer (Paszkowski et al., EMBO J. 3:2717-2722 (1984);), and ballistic particle acceleration using devices available from Agracetus, Inc., Madison, Wisconsin and Dupont, Inc., Wilmington, Delaware (see, for example, Sanford et al., U.S. Patent 4,945,050;). *Agrobacterium*-mediated transformation is generally ineffective for monocotyledonous plants for which the other methods mentioned above are preferred.

Successfully transformed cells, i.e. cells that contain a DNA construct of the present invention, can be identified by well known techniques. For example, one selection technique involves incorporating into the expression vector a DNA sequence (marker) that codes for a selectable trait in the transformed cell. These markers include dihydrofolate reductase, G418 or neomycin resistance for eukaryotic cell culture, and tetracyclin, kanamycin or ampicillin resistance genes for culturing in *E.coli* and other bacteria. Alternatively, the gene for such selectable trait can be on another vector, which is used to co-transform the desired host cell.

The marker gene can be use to identify transformants but it is desirable to determine which of the cells contain recombinant DNA molecules and which contain self-ligated vector molecules. This can be achieved by using a cloning vector where insertion of a DNA fragment destroys the integrity of one of the genes present on the molecule. Recombinants can therefore be identified because of loss of function of that gene.

Another method of identifying successfully transformed cells involves growing the cells resulting form the introduction of an expression construct of the present invention to produce the polypeptide of the invention. Cells can be harvested and lysed and their DNA content examined for the presence of the DNA using a method such as that described by Southern (1975) J. Mol. Biol. 98, 503 or Berent et al (1985) Biotech. 3, 208. Alternatively, the presence of the protein in the supernatant can be detected using antibodies as described below.

In addition to directly assaying for the presence of recombinant DNA, successful transformation can be confirmed by well known immunological methods when the recombinant DNA is capable of directing the expression of the protein. For example, cells successfully transformed with an expression vector produce proteins displaying appropriate antigenicity. Samples of cells suspected of being transformed are harvested and assayed for the protein using suitable antibodies.

Those skilled in the art will appreciate that stable and unstable (transient) transformants may be produced by plant transformation techniques. Transient transformants only transiently express the product comprising the compound of the invention encoded by the DNA construct. Transient expression systems can be useful for molecular genetic studies as well as for some specific commercial applications wherein the transformed cells that are responsible for the production of a valuable protein are harvested shortly after the transformation.

Stable transformants may be produced when the heterologous DNA sequence integrates into the genome of the host. With regard to plants the heterologous DNA may be inserted into one of the chromosomes or into the organelle genomes (mitochondrion, chloroplast).

Those skilled in the art will appreciate that *E. coli* may be used as an intermediate host and may be used in the construction of various plasmids which comprise the coding sequence using standard or modified plasmid vectors. Plant transformation could be achieved using the plasmid DNA recovered from this intermediate host and used for direct transformation of cells, for example *via* a biolistic device. Alternatively the chimeric DNA construct containing the coding sequence could be ligated into a Ti or Ri plasmid based vector for propagation in *Agrobacterium tumefaciens* or *Agrobacterium rhizogenes* and subsequent transformation into plant cells *via Agrobacterium* mediated gene transfer.

Examples of vectors include cloning vectors, expression vectors and shuttle vectors. Cloning vectors include agents that are used to carry the fragment of DNA into a recipient for the purposes of producing more of a DNA sequence. Expression vectors include agents that carry the DNA sequence into a host and directs therein the synthesis of a specific product, such as a protein or antisense transcript. An expression vector may be produced by insertion of the coding DNA sequence into an expression cassette containing an insertion site in the vector. Shuttle vectors include a genetic element that is constructed to have origins of replication for two hosts so that it can be used to carry a foreign sequence to more than one host. For example, the shuttle vector may have origins of replication for *E. coli* and *A. tumefaciens.*

Generally, the DNA is inserted into a vector in proper orientation and correct reading frame for expression. If necessary, the DNA may be linked to the appropriate trancriptional and translational regulatory control nucleotide sequences recognised by the desired host, although such controls are generally available in the vector. Regulatory elements may be derived from a plant or from an alternative source, including plant viruses or the Ti/Ri plasmid of *Agrobacterium.*

The DNA insert may be operatively linked to an appropriate promoter, for example a plant viral promoter or a plant promoter. Preferable promoters include constitutive, inducible, temporally regulated, developmentally regulated, cell-preferred and/or cell-specific promoters, tissue-preferred and/or tissue-specific promoters, and chemically regulated promoters. The promoter may also be a synthetic or artificial promoter constructed from artificial combinations of transcription factor binding sites.

Constitutive promoters include the CaMV 35S and 19S promoters (Fraley et al., US Patent No. 5,352,605). The promoter expression cassettes described by McElroy et al., Mol. Gen. Genet 231, 150-160 (1991) can be easily modified for the expression of the coding sequence and are particularly suitable for use in monocotyledonous hosts.

Yet another preferred constitutive promoter is derived from ubiquitin, which is another gene product known to accumulate in many cell types. The ubiquitin promoter has been cloned from several species for use in transgenic plants (e.g. Binet et al., Plant Science 79, 87-94 (1991).

Inducible promoters include promoters which are responsive to abiotic and biotic environmental stimuli. Abiotic environmental stimuli include light, temperature and water availability. Biotic environmental stimuli include pathogens, (including viral induced, bacterial induced, fungal induced, insect induced, and nematode induced promoters), interactions with symbionts and herbivores. Promoters may also be responsive to movement, touch, tissue damage and phytohormones (including abscissic acid, cytokinins, auxins, giberellins, ethylene, brassinosteroids and peptides such as systemin and nodulation factors).

Temporally regulated promoters include circadian regulated promoters as well as those which respond to non-circadian time-keeping mechanisms. Developmentally regulated promoters include tissue specific and cell type specific promoters for organs and other structures, including leaves, stems, roots, flowers, seeds, embryos, pollen and ovules.

Tissue-specific or tissue-preferential promoters useful for the expression of the coding sequence in plants, particularly maize and sugar beet, are those which direct expression in root, pith, leaf or pollen. Examples are the TUB1 promoter from *Arabidopsis thaliana* b1-tubulin gene (Snustad et al., Plant Cell 4, 549, 1992), the PsMT_{A} promoter region from the methallothionine-like gene of *Pisum sativum* (Evans et al., FEBS Letters 262, 29, 1990), the RPL16A and ARSK1 promoters from *A. thaliana* and further promoters disclosed in WO 97/20057 and WO 93/07278. Further, chemically inducible promoters are useful for directing the expression and are also preferred (see WO 95/19443).

Particularly preferred is the 16S rRNA, psbA and rbcL promoter.

In addition to promoters, a variety of transcriptional terminators may be incorporated into the DNA constructs of the present invention. Transcriptional terminators are responsible for the termination of transcription beyond the transgene and its correct polyadenylation. The transcriptional terminator may be derived from the same gene as the promoter or may be derived from a different gene. In a preferred embodiment, the coding sequence is operably linked to its naturally occuring polyadenylation signal sequence. Appropriate transcriptional terminators and those which are known to function in plants include the CaMV 35S terminator, the tml terminator, the pea rbcS E9 terminator and others known in the art. Convenient termination regions are also available from the Ti-plasmid of A. *tumefaciens,* such as the octopine synthase and nopaline synthase termination regions. See for example, Rosenberg et al., Gene, 56, 125 (1987); Guerineau et al., Mol. Gen. Genet., 262, 141-144 (1991); Proudfoot, Cell, 64, 671-674 (1991).

In addition to the above, the DNA construct of the present invention may comprise any other sequence that can modulate expression levels. Numerous sequences have been found to enhance gene expression from within the transcriptional unit and these sequences can be used in conjunction with a coding sequence to increase expression in transgenic plants. Various intron sequences have been shown to enhance expression, particularly in monocotyledonous cells. For example, the introns of the maize Adh1 gene have been found to significantly enhance the expression of the wild-type gene under its cognate promoter when introduced into maize cells (Callis et al., Genes Develop. 1, 1183-1200 (1987)). Intron sequences are routinely incorporated into plant transformation vectors, typically within the non-translated leader.

The constructs can also include a regulator such as a chloroplast localisation signal, chloroplast specific promoters, chloroplast specifc sequence homologues to drive homologous recombination, nuclear localization signals (Lassner et al., Plant Molecular Biology 17, 229-234 (1991)), plant translational consensus sequence (Joshi, C.P., Nucleic Acids Research 15, 6643-6653 (1987)), an intron (Luehrsen and Walbot, Mol. Gen. Genet. 225, 81-93 (1991)), and the like, operatively associated with the appropriate nucleotide sequence.

Plant transformation vectors commonly used are *Agrobacterium* vectors, which deliver the DNA by infection. Other vectors include ballistic vectors and vectors suitable for DNA-mediated transformation. These methods are known to those skilled in the art. See, for example, the review by C.P. Lichtenstein and S. L. Fuller, "Vectors for the genetic engineering of plants", Genetic Engineering, ed. P. W. J. Rigby, vol. 6, 104-171 (Academic Press Ltd. 1987).

The method of the first aspect of the invention may be used to capture carbon dioxide. Air can be used for this, although it is preferred if the air is enriched with carbon dioxide, for example it may contain up to 10% carbon dioxide. In addition, to allowing for more efficient carbon dioxide capture, it will allow for further production of biomass by virtue of additional carbon being made available to the plant cells.

Carbon dioxide capture can be achieved by providing air containing carbon dioxide to the temporary immersion bioreactor. The source of carbon dioxide may be from any source including atmospheric carbon dioxide, a carbon dioxide canister, the exhaust gas of a power plant or the exhaust gas of a combustion and/or a fermentation chamber.

The carbon dioxide concentration may advantageously be controlled in order to regulate the pH of growth medium and the leafy biomass growth in the temporary immersion bioreactor.

Biofuels may be produced by a method having the steps of : growing the leafy biomass in the temporary immersion bioreactors described above, for example the bioreactor being one or more closed temporary immersion bioreactors; harvesting the leafy biomass in a continuous, semi-continuous or batch mode process; and converting lipids or carbohydrates from the leafy biomass into a biofuel. The lipids or carbohydrates may be extracted from the leafy biomass either before or as part of the process of conversion into biofuel. The lipids or carbohydrates may alternatively be secreted into the culture medium by the leafy biomass and harvested from the culture medium for conversion to a biofuel.

In order to improve the production of biofuel by the leafy biomass, the biomass may be subjected to an environmental stress, or a combination of several stresses, to increase lipid and or carbohydrate production. The leafy biomass may also be genetically engineered in order to improve the production and accessiblity (e.g. by promoting secretion into the culture medium) of the lipid or carbohydrate that will be converted to biofuel.

Biodiesel may be produced from oils/lipids by the process of transesterification and is a liquid similar in composition to fossil/mineral diesel. Its chemical name is fatty acid methyl (or ethyl) ester (FAME). Oils are mixed with sodium hydroxide and methanol (or ethanol) and the chemical reaction produces biodiesel (FAME) and glycerol.

Bioalcohol compounds are biologically produced alcohols, most commonly ethanol (bioethanol), and less commonly propanol and butanol, and are produced by the action of microorganisms and enzymes through the fermentation of sugars, starches, or cellulose.

A second aspect of the invention provides a method of producing a polypeptide in plant cells *in vitro* comprising:
providing undifferentiated plant cells containing chloroplasts that carry a transgenic nucleic acid molecule encoding the polypeptide, wherein the plant cells display homoplastomy; and
propagating the cells according to the method of the first aspect of the invention to produce leafy biomass containing the polypeptide,

In other words, the cells are propagated by a method comprising providing undifferentiated plant cells, contacting them with an agent that promotes differentiation of the cells into leafy tissue and growing the cells in a temporary liquid immersion culture system.

Transgenic nucleic acid molecules can be introduced into chloroplasts using methods described above and in the Examples.

By homoplastomy we mean the situation where most or all of the multiple copies of the chloroplast DNA in each chloroplast of a plant cell are transformed. Homoplastomy is achieved by subculturing the transplastomic material several times, on media containing a selective agent. The selective agent is associated with a selectable marker used in the transformation construct, and can be any appropriate selectable marker, for example a resistance gene for antibiotics, such as spectinomycin or kanamycin.

Achievement of homoplastomy is standardly verified using Southern blotting.

The step of providing the undifferentiated cells where the plant is not a transgenic plant comprises:
placing cut pieces of a plant (plant material) in contact with plant hormones, the plant hormones then generate calli at the edge of those cut pieces;
the callus is then subcultured as a callus and maintained without any selection.

The cut plant material may be all or part of a root, leaf, stem, flower or seed.

The step of providing the undifferentiated cells where the plant is is a transplastomic plant comprises:
introducing the transgenic nucleic acid molecule into a chloroplast of a plant cell by a method of homologous recombination targeted to chloroplast DNA.
inducing the plant cell containing the transgenic nucleic acid molecule to form a callus of undifferentiated cells; and
propagating the callus under conditions effective to achieve homoplastomy.

The transgenic construct should contain at two least nucleic acid sequences similar (e.g. above 85% identity) to the targeted chloroplast DNA so as to achieve homologous recombination (the so-called right and left borders); a selectable marker gene and an encoded peptide or polypeptide sequence,;

Regarding the use of transplastomic undifferentiated cells, homoplastomy is achieved using antibiotic selection, for example selection with, streptomycin spectinomycin or kanamycin.

Callus homoplastomy can be achieved by various methods well known to the skilled person including, but not limited to:
(i) the nucleic acid is introduced into the chloroplast DNA of a leaf, and a plant is regenerated (grown), this plant will be subcultured (being cut, placed onto selective media to regenerate shoots from the leaf parts) at least 2 times to reach homoplastomy. When homoplastomy has been detected, for example by Southern blotting, the selected plant is transferred to a new media to produce roots, and finally to soil until it produces flowers and seeds. The seeds are then sowed onto a selective media, and the arising shoots are used to generate calli.
(ii) the method of (i) above but without transferring the plant to soil and flowering. As soon as the homoplastomy has been reached, the leaves of the plant are used to generate the calli.
(iii) the nucleic acid is introduced into the chloroplast DNA of a leaf, and as the first leaf appeared, a callus is induced on selective media (this plant material is heteroplastomic, because it contains a mixture of transformed and non transformed chloroplast DNA, which can be verified by Southern blot), and the callus is subcultured as a calli on selective media until it subsequently reaches homoplastomy.
(iv) the nucleic acid is introduced into the chloroplast DNA of undifferentiated cells, and transplastomic calli are subcultured as a calli onto selective media until reaching homoplastomy.

Preferably, the nucleic acid molecule comprises a selectable marker gene. Typically, the selectable marker gene is an antibiotic resistance gene such as aadA, nptll, AphVI.

Typically, the nucleic acid molecule is inserted into a vector or a PCR fragment.

Typically, the vector is a plasmid, and typically it can be propagated in *Escherichia coli,* yeast, insect or mammalian cells. Preferably, the plasmid is a chloroplast transformation plasmid.

It is preferred if the expression of the polypeptide is driven by a strong chloroplast specific promoter. Suitable promoters include a 16S rRNA promoter, a psbA promoter and a rbcL promter.

Methods of plant cell and chloroplast transformation are well know to the skilled person and include transgenic methods as discussed above and as described in Sambrook and Russell (2001), Molecular Cloning, A laboratory manual; Grierson and Covey (1988) Plant molecular biology and Watson et al. (1997) Recombinant DNA.

The amount of light available and/or the amount of sucrose available in the growth medium may influence the production of the polypeptide. The growth media and conditions including the gas mixture (e.g. carbon dioxide concentration) can be readily optimised by a skilled person for the production of each specific polypeptide based on the plant material being used and the biomass required to be produced.

The method of the second aspect of the invention preferably includes the further step of obtaining the polypeptide from the leafy biomass. Also disclosed herein is a polypeptide so-obtained by the method of the second aspect of the invention. Conveniently, the polypeptide is obtained by crushing the leafy tissue to produce a tissue extract and isolating the polypeptide from the tissue extract.

Conveniently, the polypeptide is purified from the tissue extract using at least one of filtration, HPLC, ion exchange resin extraction, hydrophobic interaction resin extraction, affinity chromatography or oil-water phase separation.

The polypeptide may comprise a tag for use in purifying the polypeptide. The tag may be a cleavable or non-cleavable tag, such as any one of a GST, biotin, 6His, Strep, HA or myc tag.

Also disclosed herein is leafy biomass obtained by method of the first aspect of the invention.

The polypeptide obtained from the method may be any one of a therapeutic polypeptide, an enzyme, a growth factor, an immunoglobulin, a hormone, a structural protein, a protein involved in stress responses of a plant, a biopharmaceutical or a vaccine antigen

A third aspect of the invention provides a method for obtaining a component present in leafy biomass, the method comprising producing leafy biomass according to the first aspect of the invention and obtaining the component from the leafy biomass. Typically, the component is obtained in a substantially pure form, and so the method may comprise the further step of purifying the component. The substantially pure form typically contains >90%, or >95% or >99% of the component.

The component may be obtained by its secretion from the leafy biomass or by extraction from the leafy biomass, for example by crushing the leafy biomass to release the component.

The component obtained may be a medicinal product, a recombinantly expressed polypeptide, a carbohydrate, a lipid, an oil, a volatile aromatic compound, an anti-oxidants, a pigment, a flavour or flavour precursor; and the component may be either endogenous or exogenous.

The invention further provides for the processing of the component obtained into a further product, for example a biofuel, food stuff or medicinal product.

Also disclosed herein is a system for producing a polypeptide in plant cells *in vitro* comprising:
an agent which promotes differentiation of undifferentiated cells into leafy tissue; and a
nucleic acid molecule encoding the polypeptide, which is adapted for introduction into and expression in chloroplasts.

In a further aspect of the invention there is provided a method of capturing carbon dioxide, the method comprising carrying out the method of the first aspect of the invention.

Also disclosed herein but not claimed is a method of purifying a sample comprising exposing the sample to be purified to the leafy biomass derived from the method of the first aspect of the invention.

The purification process may be to remove one or more toxins.

Also disclosed herein but not claimed is a method of manufacturing a pharmaceutical composition comprising formulating:
a component obtained by the methods of the other aspects of the invention and a pharmaceutically acceptable carrier diluent, excipient or carrier.

Furthermore, also disclosed herein is a pharmaceutical product comprising a component obtained by the methods of the aspects of the invention and a pharmaceutically acceptable carrier diluent, excipient or carrier.

Also disclosed herein but not claimed is a method of manufacturing a biofuel comprising fermentation or transesterification of a component obtained by the methods of the aspects of the invention. Also disclosed herein is a biofuel obtained by the above disclosed method of manufacture.

The present invention will now be described in more detail with reference to the following non limiting Examples and Figures.

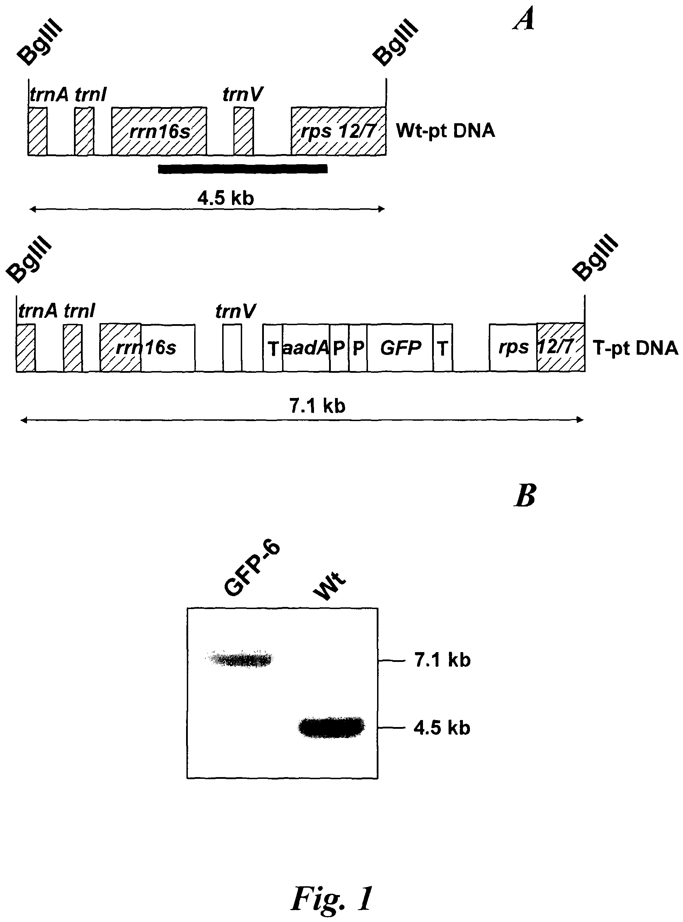

**Figure 1****. Southern blot analysis of the transplastomic GFP-6 line.**
   (A) Physical map of wild-type *Nicotiana tabacum* petit Havana (Wt-pt DNA) and transformed (T-pt DNA) tobacco plastome in the targeted chloroplast region. Arrows below each map indicate the predicted DNA fragment sizes after BgIII digestion of respective genomic DNA. Vector sequence is indicated in white, whereas the tobacco plastome sequence is in orange. (B) Southern blot analysis after digestion of the total genomic DNA with BgIII for the transgenic line GFP-6 (GFP-6) and wild-type tobacco. Digested genomic DNA was run on a 0.7% (w/v) agarose gel, transferred onto a nylon membrane and probed with Dig-labelled PCR fragment corresponding to the amplification of the targeted region with primers PHK40-F and rps12-out-R (black bar).

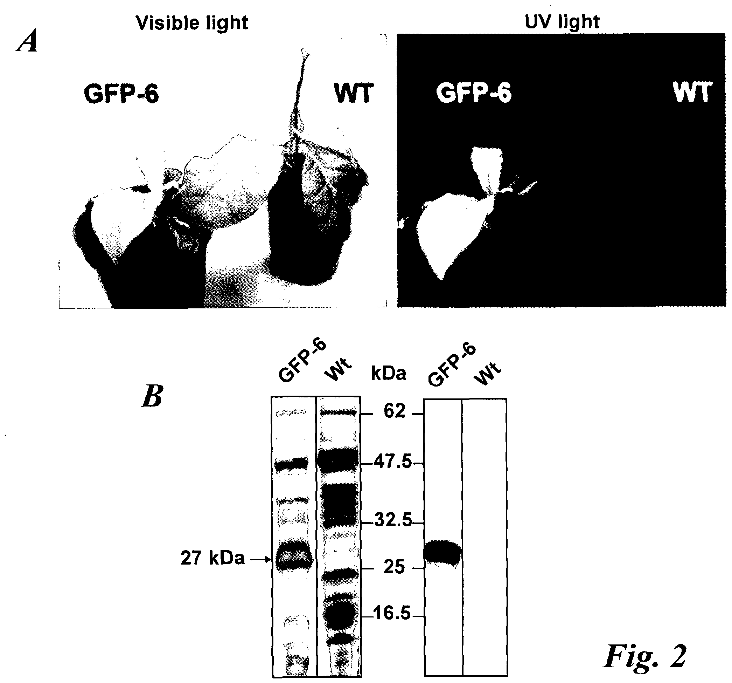

**Figure 2****. GFP+ detection in transplastomic GFP-6 tobacco line.**
   GFP expression was (A) visualised in the GFP-6 homoplastomic line (GFP-6) under UV and visible light along with control wild-type (wt) tobacco plant. (B) Protein electrophoresis of soluble proteins from GFP-6 and Wt lines. 5 µg of total soluble protein extract of each plant were loaded onto a 12.5% (w/v) SDS-PAGE gel along with prestained protein marker (New England Biolabs, UK) and protein separation was visualised by silver staining. GFP was specifically detected by Western blotting using a specific anti-GFP antibody. Migration of prestained markers is also indicated.

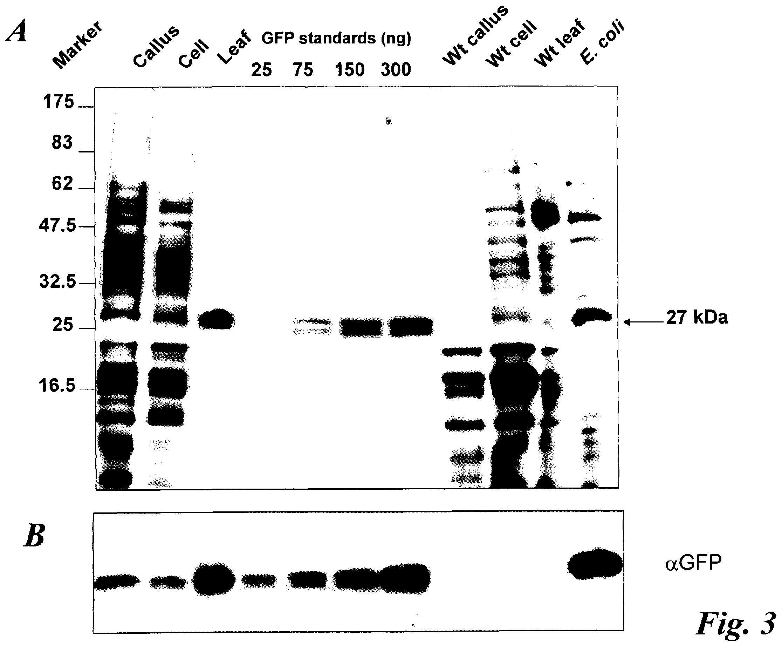

**Figure 3****. GFP+ expression in different transplastomic tobacco tissues.**
   Total soluble protein extracts from calli, cell suspensions and leaves from GFP-6 and wild-type tobacco were generated. For calli and cell suspensions, 5 µg total soluble protein were loaded per lane onto a 12.5% (w/v) SDS-PAGE gel whereas only 1 µg was loaded for leaves extracts. (A) corresponds to the silver-stained gel, whereas (B) represents the corresponding Western blot using a GFP antibody. GFP standards were purchased from Roche Life Science, UK and the Prestained Protein Marker from New England Biolabs, UK. The ladder size of the marker proteins are in kDa. Wt stands for *Nicotiana tabacum* Petit Havana, and *E. coli* corresponds to the protein extraction from an *E. coli* KRX strain transformed with pFMGFP.

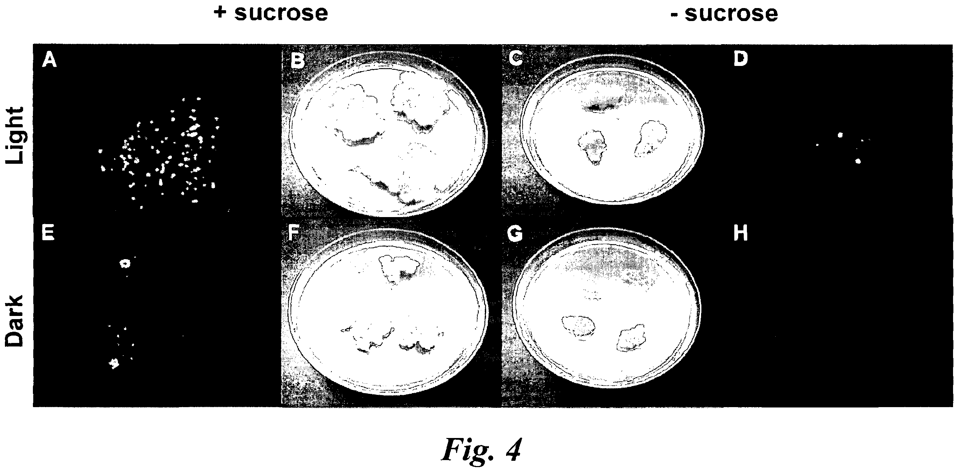

**Figure 4****. Growth of GFP-6 transplastomic calli under different conditions.**
   Pictures of homoplastomic calli GFP-6 were taken after 4 weeks of growth at 25°C. Plates (A, B, C and D) were grown with 16/8h light with similar intensity as for tobacco seedlings and (E, F, G and H) were grown in the dark. Only A, B, E and F contained 3% (w/v) sucrose in the media. All media contained 500 mg/L spectinomycin and 500 mg/L streptomycin. Fluorescence emission was detected at 520 nm following excitation at 490 nm using an Axiovert 200 M inverted microscope (Carl Zeiss, Goettingen, Germany) along with the Axiovision software (Version 3.0). Fluorescent exposure was 30 ms, 100 ms and 600 ms for A, D and E, H respectively. Microscope magnification was the same in A, D, E and H at 40x.

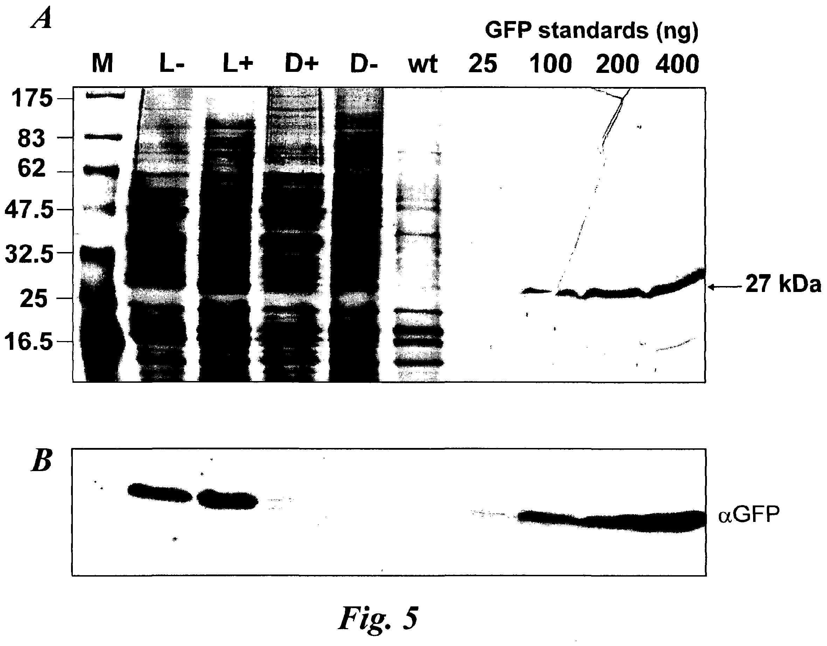

**Figure 5****. Detection of GFP+ in GFP-6 calli grown under different conditions.**
   Total soluble protein were extracted from light (L) or dark (D) grown calli as well as wild-type (Wt) grown under light and sugar. Presence of sucrose in media is indicated by (+) whereas sucrose-free media is described with (-). 5 µg of total soluble protein of the respective calli were loaded onto a 12.5% (w/v) SDS-PAGE gel (L-, L+, D+, D-, wt) and total protein content (A) was detected by silver staining. M represents the Prestained Protein Marker (New England Biolabs, UK) and corresponding sizes are indicated on the left in kDa. (B) GFP+ presence was specifically detected with an anti-GFP antibody. GFP standards (Upstate, USA) were added in the quantities indicated in nanograms.

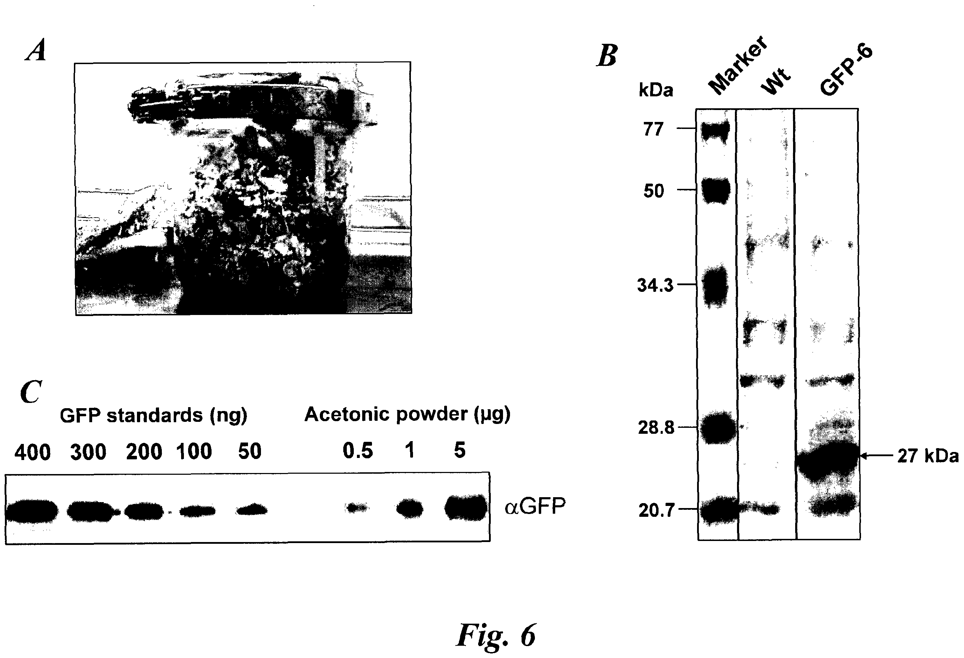

**Figure 6****. GFP+ expression in newly formed green biomass from a temporary immersion bioreactor.**
   After a 6-weeks incubation period, tobacco biomass of the GFP-6 line (A) was removed from the temporary immersion bioreactor. Total proteins were extracted from newly formed leaves using the acetone extraction protocol and loaded (B) onto a 10% (w/v) SDS-PAGE gel along with prestained SDS-PAGE standard low range (Bio-Rad Laboratories, UK). Proteins from wild-type (wt) and GFP-6 line (GFP-6) were visualised with Coomassie Blue staining. Different dilutions of acetonic powder were analysed by immunoblotting (C) with an anti-GFP antibody and compared to known quantity of GFP protein (Upstate, USA).

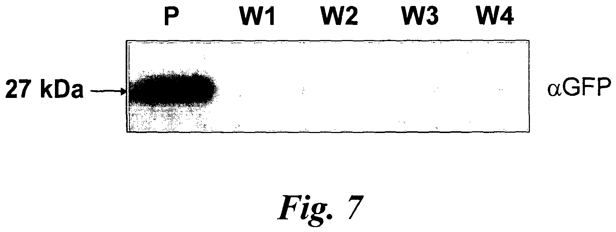

**Figure 7****. GFP detection during the acetone precipitation protocol.**
   Western blot representing the GFP presence in several samples from different steps of the acetone extraction protocol. Pellets were resuspended directly in the loading buffer whereas washes were dried overnight in a speedvac (Savant, NY, USA) before addition of the loading buffer. Only 5 µl of pellet (P) sample were loaded while all supernatants from washes (W) 1 to 4 were added.

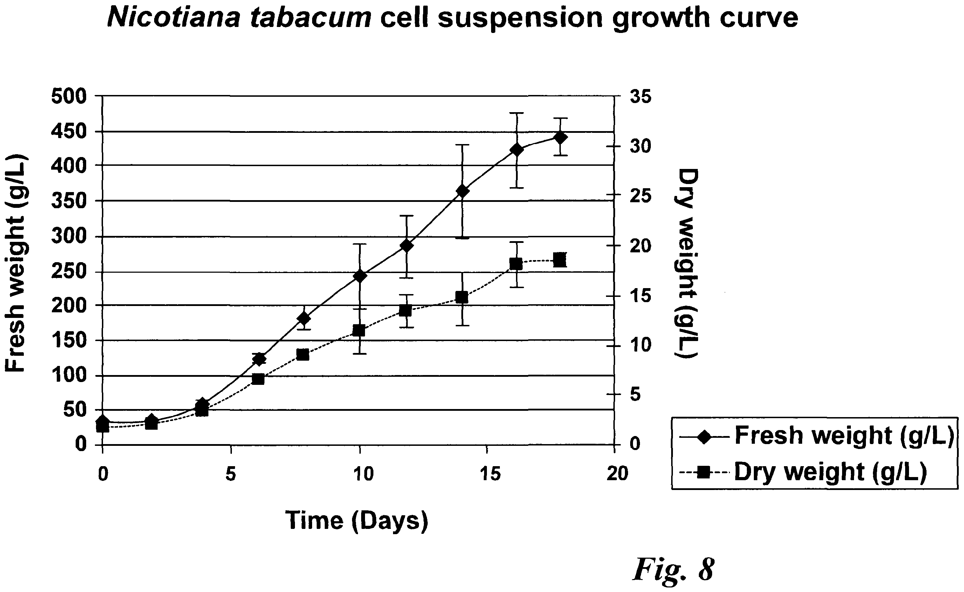

Figure 8. Dry and fresh weight of *Nicotiana tabacum* Petit Havana cell suspensions.
   Fresh and dry weights of tobacco wild-type cells were determined every 2 days during a 18 day-growth period. Dry weight was measured after leaving fresh tobacco cells 24 h at 80°C. Measurements were done in triplicate.

### Example 1: Contained and high-level production of recombinant protein in plant chloroplasts using a temporary immersion bioreactor

### Summary

Chloroplast transformation is a promising approach for the commercial production of recombinant proteins in plants. However, gene containment still remains an issue for the large-scale cultivation of transplastomic plants in the field. Here we have evaluated the potential of using tobacco transplastomic cell suspensions for the fully contained production of a model protein, a modified form of the green fluorescent protein (GFP+). In transplastomic leaves GFP+ expression reached approximately 60% of total soluble protein (TSP). Expression in cell suspension cultures (and calli) was much less (1.5% of TSP) but still produced about 7.2 mg per litre of liquid culture. We further investigated the different factors influencing GFP+ production in calli and highlighted the importance of light as an input. Finally we describe the development of a novel protein production platform in which transgenic cell suspension cultures were placed in a temporary immersion bioreactor in the presence of Thidiazuron to initiate shoot formation. GFP+ yield reached an impressive 660 mg per L of bioreactor. This new production platform, combining the rapid generation of transplastomic cell suspension cultures and the use of temporary immersion bioreactors, is a promised route for the fully-contained low-cost production of recombinant proteins.

### Results

### Generation of homoplastomic tobacco shoots expressing GFP+

The vector that was constructed to express GFP+ in tobacco chloroplasts is derived from pJST10, which was used to express TetC antigen in tobacco chloroplasts (Tregoning *et al,* 2003). Plasmid pJST10 targets the insertion of the expression and selection cassette between tobacco chloroplast genes *rrn16S* and *rps12*/*7* (Figure 1A). After bombardment, several spectinomycin-resistant shoots were produced from 10 independent bombardments and *gfp+* integration was detected by PCR analysis in 4 shoots out of 6 analysed (data not shown). GFP-6 was selected for further experiments and submitted to 4 rounds of subculture on MS selective media.

To confirm that all chloroplasts of the GFP-6 line were transformed, total genomic DNA was extracted from a leaf of this plant, digested with BgIII and subjected to Southern blot analysis (Figure 1). As expected a probe corresponding to the insertion site hybridised to a single band of 4.5 kb in the wild-type tobacco DNA. In contrast a 7.1-kb band was detected in the GFP-6 line which is consistent with insertion of the *gfp+* gene and selectable marker. The lack of the 4.5 kb band in GFP-6 also indicated that GFP-6 was homoplastomic (Figure 1 B).

### GFP+ expression in the GFP-6 line

The tobacco GFP-6 line was grown on soil and expression of GFP+ tested by exposing plants to a UV/blue light source (Figure 2A). A strong green fluorescence could be observed in GFP-6 but not in wild-type, indicating GFP+ expression in GFP-6. To confirm accumulation of GFP, total soluble proteins were extracted from the GFP-6 and wild-type lines and separated on a SDS-PAGE gel (Figure 2B). An immunoblotting analysis using a specific anti-GFP antibody confirmed the accumulation of GFP+ and the lack of significant break-down products. Analysis of a silver-stained (Figure 2B) and Coomassie-blue stained gels (data not shown) revealed that GFP+, migrating at 27 kDa, was highly expressed and the dominant protein in the soluble extract.

### Comparison of expression levels in leaves, calli and cell suspensions of the GFP-6 tobacco line

The T0 seeds obtained from the GFP-6 line were germinated on MS plates *in vitro* and the resulting young leaves were used to generate corresponding transplastomic calli and cell suspensions. GFP+ expression was evaluated in the callus state, cell suspension culture and in leaves of the parental plant GFP-6 by SDS-PAGE (Figure 3A) and semiquantitative immunoblotting analysis (Figure 3B) using known amounts of commercially available GFP as standards.

The most striking result of this comparison was the extremely high level of GFP+ expression within tobacco leaves (Figure 3A) compared to the calli and cell suspensions. The immunoblots indicated that GFP+ expression in leaves was about 60% of TSP, which was equivalent to about 5 mg/g fresh weight, whereas expression in callus and cell suspensions was about 1.5% of TSP (Figure 3B). After taking into account the growth of the cell suspensions (Supplementary Figure 8), the rate of GFP+ production in transplastomic cell suspensions was estimated to be approximately 0.4 mg/Uday.

### Influence of light and sugar on GFP expression in calli

In order to assess the importance of light and exogenous sucrose on GFP+ expression, transplastomic calli from the GFP-6 line were grown for one month on Callus Induction Media (CIM) either with or without light and with or without sucrose (Figure 4), but in the presence of 500 mg/L of spectinomycin to maintain selection. As seen in Figure 4, calli growth was significantly promoted by the addition of sucrose, independent of the light intensity. When both light and sugar were available to the transplastomic calli, a large number of small chloroplasts/plastids expressing GFP+ could be identified, which were dispersed within the cytosol (Figure 4A). If light or sugar were not supplied to the calli, GFP fluorescence decreased and the number of chloroplasts/plastids declined and localised to the centre of the cell (Figure 4D and 4E). No GFP expression was detected in calli grown in the absence of light and sugar (Figure 4H).

Immunoblotting experiments confirmed that cells grown in complete darkness expressed little or no GFP, whereas in the light, regardless of the presence or absence of sucrose, expression went up (Figure 5).

When grown in the presence of light and sucrose (L+), the level of GFP+ expression was estimated by immunoblotting to be about 4% of TSP (Figure 5). When normalised to the fresh and dry weights of calli, this corresponded to a GFP+ expression level of up to 48 µg/g f.w. (fresh weight) or about 1 mg/g d.w. (dry weight), respectively.

### Use of temporary immersion bioreactors for the production of transplastomic biomass

Given that transplastomic gene expression seemed to be highest in leaf tissue we sought to develop a method for the rapid production of leaf tissue from callus/cell suspensions. In preliminary experiments, we found that addition of Thidiazuron (TDZ), which is known to promote somatic embryo growth in tobacco (Gill and Saxena, 1993), was able to induce shoot formation from GFP-6 calli grown on solid MS medium (data not shown). In order to scale up the production capacity, transplastomic cell suspensions from the tobacco GFP-6 line were loaded into a 2-L bioreactor and temporally submerged in MS media supplemented with 0.1 µM TDZ. After about six weeks, a large number of shoots were produced (Figure 6A). During the first 14 days, no growth could be detected and shoots only started to grow after this period. Possibly this lag period is related to the time needed for cells to redifferentiate from callus tissue to leafy tissue in tobacco in a similar manner to the observed switch between calli and meristematic tissues in *Arabidopsis thaliana* (Gordon *et al,* 2007).

After 40 days, the total biomass was removed from the bioreactor for analysis. Inspection of the plant material revealed the presence of mainly healthy leaves with minimal vitrification.

A total amount of about 470 g of fresh weight biomass was produced in the 2-L bioreactor. To evaluate the amount of GFP+ produced within this biomass, a protein precipitation protocol was developed based on protein precipitation in acetone. Using this method, a powder was produced, weighed and loaded onto a SDS-PAGE gel to detect produced GFP+ (Figure 6B). A clear band, absent from the wild type, and with a size of about 27 kDa was detected. To quantify the production of GFP+ within the transplastomic biomass, several amount of acetonic powder were loaded and 1 µg of this powder was estimated to contain approximately 150 ng of GFP+ by immunoblotting (Figure 6C). This indicated that the expression level reached about 2.8 mg/g fresh weight.

In the bioreactor, total GFP production reached about 660 mg/L at an approximate rate of 17 mg/Uday of GFP over the 40-day growth period. This value is approximately 42-times higher than the rate potentially achievable with cell suspensions of 0.4 mg/L/day.

### Discussion

### Tobacco transplastomic cell suspension cultures

Most work so far in the chloroplast transformation sphere has focussed on leaves for the expression of several genes of interest. Some work has been done on expression in transplastomic potato tubers (Sidorov *et al,* 1999) and transplastomic tomato fruit (Ruf *et al,* 2001) but the expression yields were relatively poor (0.05 and 0.5% of TSP respectively). However, planting transgenic plants, even if they are transplastomic, could still be badly perceived by a large part of the public and the possible environmental issues could have a drastic impact on any future developments. In addition, there are very significant regulatory costs associated with each new transplastomic field releases. Recombinant protein production in contained transplastomic cell based cultures would overcome many of these concerns and should significantly reduce regulatory costs due to the highly contained nature of this new production system.

To compare different types of expression system, we first created a homoplastomic line of tobacco that expressed a variant of Green Fluorescent Protein (GFP+). GFP has previously been shown capable of high expression in chloroplasts in a range of different plants including tobacco (Khan and Maliga, 1999; Newell *et al*, 2003), potato (Sidorov *et al,* 1999) and lettuce (Kanamoto *et al,* 2006). The levels of GFP expression described here, approx 60% of TSP in leaves, is at the high end of expression and is similar to the value observed for GFP expression in lettuce where GFP at 36% of TSP was achieved (Kanamoto *et al,* 2006).

Our results showed clearly that levels of GFP+ expression are less in calli and cell suspension cultures compared to leaves (Figure 3 and 5) with levels varying between 1.5 to 4% of TSP, which is similar to the expression of GFP in transient transplastomic lettuce calli of 1 % of TSP (Lelivelt *et al,* 2005).

Expression of GFP+ in transplastomic cell suspensions reached about 1.5% of TSP, which corresponds to 7.2 mg/L at a production rate of 0.4 mg/Uday (Figure 5). This expression level could possibly be increased by optimisation of the culture media e.g. by the addition of polyvinyl pyrrolidone and/or gelatine, which have helped improve yields of protein expression in nuclear transformed plant cells (Kwon *et al,* 2003; Lee *et al,* 2002). We as well showed that GFP+ level could be increased to about 4% TSP when light and sugar content were better optimised (Figure 5). If this result is extrapolated to the cell suspensions growth period, GFP+ production could potentially reach about 1 mg/Uday.

### Factors influencing the production of GFP+ in transplastomic calli

The generally lower expression levels in transplastomic calli and cell suspensions might directly be explained by the choice of the chloroplast transformation vector and specifically by the respective promoter that drove the GFP+ expression. Prrn, the promoter of the *RNA16S* gene used in pFMGFP, is similar to the *RNA16S* promoter from rice, whose activity decreased 7 fold in rice embryogenic cells in comparison to its activity in leaves (Silhavy and Maliga, 1998). The same phenomenon might have occurred here since the cell suspension plastids are less differentiated than the leaf chloroplasts. However, further work will have to assess GFP mRNA levels in both leaves and calli to be able to differentiate between a reduction in mRNA levels or a possible variation in chloroplast numbers.

Light seemed to be obviously indispensable for significant GFP+ expression (Figure 5), whereas sucrose appeared more related to increased cell growth. However, these results might be biased, because despite a 1-month incubation period in the dark, GFP+ is very stable and the expression detected in callus grown on sucrose-supplemented media in the dark could correspond to residual GFP+ production from the tobacco cells before they had been transferred to the dark. In fact, GFP expression, driven by the same promoter, in potato microtubers reached only 0.05% TSP (Sidorov *et al,* 1999), which might indicate the real baseline for expression of GFP under the *prrn* promoter is much less than that observed here.

In our experiments, it was noticeable that the calli and cell suspensions remained green and possessed a large number of chloroplasts widely spread around the cell (Figure 4A). GFP+ reached in these cells about 4% TSP, and such a high level might indicate that these cells are actually transient cell suspensions which are not completely dedifferentiated and in which the plastids are still similar to functional chloroplasts.

### Transplastomic biomass production in temporary immersion bioreactors

GFP+ production in leaves was vastly superior to that in undifferentiated cells (Figure 3) and therefore attempts were made to promote shoot induction from transplastomic callus tissue. The addition of thidiazuron (TDZ) to the solid media induced the formation of shoots from calli after 6 weeks (data not shown). Interestingly, the observed growth in the magenta boxes was not linear, and no particular growth was detected within the first 2 weeks.

However, when transplastomic cell suspensions were placed under temporary immersion conditions where the cell material was subjected to being submerged in liquid occasionally, for only short periods using a temporary immersion type bioreactor, the production of "leafy" material was efficient and significant, with the final biomass production being extremely abundant (Figure 6A). A similar lag phase in the biomass growth was observed for both the solid media based induction and in the temporary immersion bioreactor based induction where no growth was detected for the first 2 weeks.

The material was mainly composed of healthy small leaves and the GFP+ content was estimated to reach about 0.66 g/L (Figure 6C). These values are slightly lower than the production observed in Chinese Hamster Ovary (CHO) cells (Wilke and Katzek, 2003) but are one of the highest attained in a plant-based system. Furthermore, the expression levels were obtained without any optimisation and future developments should improve the production and scalability of the process. For example, the exchange of glass bottles for disposable bags nearly doubled the amount of coffee somatic embryo produced using temporary immersion (Ducos *et al,* 2008), possibly due to a better light penetration and repartition. If a similar system was to be used with transplastomic tobacco shoots, the production yields could reach more than 1 g/L.

The system described here, properly scaled should be much less labour intensive than the production of whole plants in a green house, and also does not require glasshouse containment facilities. It also offers a potentially faster route to the production of target protein from transformed tissue as seeds do not need to be produced. In fact, once an homoplastomic tobacco line is identified, only one month is required to obtain a cell suspension culture suitable for the temporary immersion bioreactors, whereas, if seeds need to be produced, about 3 months are necessary (Molina *et al,* 2004). A combination of the temporary immersion growth of transplastomic shoots with recently described disposable bioreactors (Terrier *et al,* 2007; Ducos *et al,* 2008) is therefore a promising route for the low-cost production of biopharmaceuticals in plants.

### Experimental procedures

### Tobacco shoots, calli and cell suspensions generation

*Nicotiana tabacum* Petit Havana (Tobacco) seedlings, calli and cell suspensions were grown at 25°C, under a 16-hour photoperiod (about 100 µmol/m²/s) at 30% humidity in a Fi-Totron 600H incubator (Sanyo, Watford, UK). Tobacco seedlings were germinated onto MS media (Murashige and Skoog, 1962) and calli were produced by placing small pieces of leaves onto Callus Induction Media (CIM), which is a MS media supplemented with 1 mg/L of 1-Napthaleneacetic acid (NAA) and 0.1 mg/L Kinetin (K). Cell suspensions were generated by incubating large amounts of calli in CIM media lacking the agar under a constant agitation of 140 rpm. All plant hormones and media were purchased from Sigma, St Louis, MO, USA.

### Construction of chloroplast transformation vector

Chloroplast transformation vector pFMGFP was created by swapping *TeTC* gene for *gfp+* gene (Scholz *et al,* 2000) in previously characterized tobacco chloroplast vector pJST10 (Tregoning *et al,* 2003) by double digestion using Ndel and Xbal restriction sites.

### Generation of transplastomic tobacco plants

Biolistic transformation of 6-weeks old wild-type tobacco leaves with tobacco chloroplast transformation vector pFMGFP was performed on RMOP media (Svab *et al,* 1990) with a composition based on MS medium supplemented with 1 mg/L thiamine, 100 mg/L myo-inositol, 1 mg/L N6-benzyladenosine (BAP) and 0.1 mg/L 1-Napthaleneacetic acid (NAA) using the PDS1000/He (Bio-Rad, Hercules, CA, USA) biolistic device with rupture disks of 1100 psi. Vector pFMGFP was coated onto 550 nm gold particles (SeaShell, La Jolla, CA, USA) according to manufacturer's recommendations. After bombardment, leaves remained in the dark for 48 hours before plant materials were cut into small pieces (5 mm x 5 mm) and placed onto RMOP media supplemented with 500 mg/L spectinomycin dihydrochloride. Spectinomycin resistant shoots were subcultured on the same media 4 times.

### Southern blot analysis

Vector integration into tobacco plastome was evaluated by PCR unsing a primer annealing at the start of *gfp*+ and the other on the tobacco plastome outside the homologous regions of the vector pFMGFP (data not shown) and transplastomic GFP-6 line was chosen for all further experiments. Homoplastomy state was evaluated by Southern hybridisation of digested total genomic DNA from both wild-type and transplastomic GFP-6 lines. About 7 µg of genomic DNA was digested with BgIII and was run on a 0.7% (w/v) agarose gel. The DNA gel was transferred by capillarity onto a nylon membrane (Hybond-N, Amersham, Uppsala, Sweden) overnight in 20 x SSC buffer.

The probe was DIG-labelled overnight at 37°C using DIG High Prime DNA Labelling and Detection Starter Kit II (Roche Applied Science, UK). A 3 kb probe homologous to the targeted region was obtained by PCR using primers pJST10-F 5' AATTCACCGCCGTATGGCTGACCGGCGA 3' and Rps12-OUT-R 5' TTCATGTTCCAATTGAACACTGTCCATT 3' and tobacco genomic DNA as template. Probe labelling and hybridisation were performed according to manufacturer's recommendations with a final probe concentration of 25 ng/ml. Specific signal detection with provided CSPD was detected by X-ray film (Amersham, Uppsala, Sweden) according to the manufacturer's guidelines. After homoplastomy confirmation by Southern blot analysis, GFP-6 plantlet was transferred to soil and allowed to produce seeds. This T₀ seeds were germinated onto MS media supplemented with 500 mg/L spectinomycin and young T₀ leaves were used for calli and cell suspensions generation.

### Protein extractions

First, total soluble protein extraction was performed according to (Kanamoto *et al,* 2006). Plant materials (leaves, calli, cell suspensions) were grounded into a fine powder with liquid nitrogen and mixed with total soluble extraction buffer (50 mM HEPES pH 7.6, 1mM DTT, 1mM EDTA, 2% (w/v) polyvinyl pyrrolidone and one tablet of complete protease inhibitors EDTA-free cocktail (Roche Products Ltd, Welwyn Garden City, UK). Plant mixtures were vortexed during 1 min and spun down at 13,000 rpm for 30 min at 4°C. Supernatants were aliquoted and stored at - 20°C until further use.

The second method was based on a total protein extraction protocol based acetone precipitation. Plant material was grounded to a fine powder in liquid nitrogen. 30 ml of extraction buffer (80% (v/v) acetone, 5 mM ascorbate) were added to 2 g of plant powder or leaf equivalent and the mixture was homogenised with an Ultra-Turrax (IKA, Heidelberg, Germany) for 15 s on ice. Proteins were pelleted by a centrifugation at 5,000 g for 5 min at 4°C. The supernatant was discarded and the pellet was washed 4 times using the same extraction buffer and same centrifugation conditions. Then the pellet was resuspended in pure acetone and homogenised again. Proteins were spun down once more at 10,000 g for 5 min at 4°C. The supernatant was discarded and the pellet was washed 3 more times in pure acetone. During the last wash, the buffer was aliquoted and dried using a Speed-Vac (Savant, Holbrook, NY, USA) and the residual powder was termed acetonic powder. The presence of GFP in the pellet and the different washes was detected by Western blot analysis (Supplementary Figure 7).

### Electrophoresis and Western blot analysis

Proteins from transplastomic and wild-type samples were resolved in 12.5% (w/v) SDS-PAGE gels along with protein markers and commercially available recombinant GFP (Upstate, Waltham, MA, USA) for quantification purposes. Protein gels were directly stained with Coomassie Blue or with silver staining.

Following electrophoresis, proteins were transferred onto a 0.2 µm nitrocellulose membrane (Bio-Rad, Hercules, CA, USA) either using the mini Trans-Blot^{®} system (Bio-Rad, Hercules, CA, USA) or by using the iBlot dry transfer system according to manufacturer's recommendation (Invitrogen, UK). After the transfer, GFP specific detection was performed with primary rabbit polyclonal anti-GFP antibody (provided by Prof Nixon, Imperial College London, UK) diluted 1:20,000 whereas the secondary antibody (Horseradish Peroxidase-conjugated goat anti-rabbit immunoglobulin G, Amersham, Uppsala, Sweden) was diluted 1:10,000. Biochemical detection was performed with the ECL SuperSignal® West Pico Chemiluminescence Substrate kit (Pierce Biotechnology Inc., UK).

### Temporary immersion bioreactors

Tobacco biomass was generated by placing about 7 grams of *Nicotiana tabacum* Petit Havana cell suspensions in a 2-L temporary immersion bioreactor (Ducos *et al,* 2007). Immersions were performed over a 40-day period with 1-L MS media supplemented with 0.1 µM Thidiazuron (TDZ, Sigma, UK) every three hours for 5 min. Additionally, the media contained 100 mg/L of spectinomycin to prevent contamination and to select for transplastomic cells. The TDZ (Thidiazuron) concentration in MS media was estimated to be optimal at 0.1 µM by researchers in Nestlé, based on calli solid induction in Petri dishes (data not shown). The medium was pushed by an air pump into the 2-L vessel for 3 min and allowed to return to the original bottle by gravity for 2 more minutes. Light conditions and temperature were similar to the calli and cell suspensions growth experiments.

### Fluorescence microscopy

Transplastomic tobacco calli and cell suspensions expressing GFP and originating from the GFP-6 line were observed using an Axiovert 200 M inverted microscope (Carl Zeiss, Goettingen, Germany) and the Axiovision software (version 3.0). Excitation and emission wavelength were set up at 491 nm and 512 nm respectively, optimal for GFP+ detection (Scholz *et al,* 2000). Exposures and magnifications varied depending on the experiment and are indicated in each figure.

### Table S1. Ratios between fresh, dry weights and acetonic powder.

These ratios were calculated for the determination of a robust quantification of GFP. Values represented an average of at least 4 repetitions for fresh weight (f.w.), dry weight (d.w.) and acetonic powder (powder). Calli and cell suspensions (Cells) were harvested at the end of their respective growth phases and leaves measurement was performed on young 2-3 weeks old plantlets (with about 4 leaves per plant, similar to the biomass produced in the temporary immersion bioreactor).

| **Tissue** | **d.w./f.w. (%)** | **Powder/d.w. (%)** |
|---|---|---|
| Leaves | 6.6 ± 0.9 | 28.3 ± 1.1 |
| Cells | 4.4 ± 0.3 | 14.1 ± 1.4 |
| Calli | 3.6 ± 0.4 | 12.4 ± 1.3 |

### References

Azhagiri AK and Maliga P (2007) Exceptional paternal inheritance of plastids in Arabidopsis suggests that low-frequency leakage of plastids via pollen may be universal in plants. Plant J 52: 817-823.
Birch RG (1997) PLANT TRANSFORMATION: Problems and Strategies for Practical Application. Annu Rev Plant Physiol Plant Mol Biol. 48:297-326.
Brinkmann N and Tebbe CC (2007) Leaf-feeding larvae of Manduca sexta (Insecta, Lepidoptera) drastically reduce copy numbers of aadA antibiotic resistance genes from transplastomic tobacco but maintain intact aadA genes in their feces. Environmental biosafety research 6: 121-133.
Daniell H, Lee SB, Panchal T and Wiebe PO (2001) Expression of the native cholera toxin B subunit gene and assembly as functional oligomers in transgenic tobacco chloroplasts. Journal of molecular biology 311: 1001-1009.
Doran PM (2000) Foreign protein production in plant tissue cultures. Current opinion in biotechnology 11: 199-204.
Ducos J, Labbe G, Lambot C and Pétiard V (2007) Pilot scale process for the production of pre-germinated somatic embryos of selected robusta ( Coffea canephora ) clones. In Vitro Cellular & Developmental Biology - Plant 43: 652-659.
Ducos J, Terrier B, Courtois D and Pétiard V (2008) Improvement of plastic-based disposable bioreactors for plant science needs. Phytochemistry Reviews 7: 607-613.
Farran I, Rio-Manterola F, Iniguez M, Garate S, Prieto J and Mingo-Castel AM (2008) High-density seedling expression system for the production of bioactive human cardiotrophin-1, a potential therapeutic cytokine, in transgenic tobacco chloroplasts. Plant biotechnology journal 6: 516-527.
Fischer R, Emans N, Schuster F, Hellwig S and Drossard J (1999) Towards molecular farming in the future: using plant-cell-suspension cultures as bioreactors. Biotechnology and applied biochemistry 30 (Pt 2): 109-112.
Fischer R, Stoger E, Schillberg S, Christou P and Twyman RM (2004) Plant-based production of biopharmaceuticals. Current opinion in plant biology 7: 152-158.
Fox JL (2003) Puzzling industry response to ProdiGene fiasco. Nature biotechnology 21: 3-4.
Gill R and Saxena PK (1993) Somatic embryogenesis in Nicotiana tabacum L.: induction by thidiazuron of direct embryo differentiation from cultured leaf discs. Plant Cell Reports 12: 154-159.
Gleba Y, Klimyuk V and Marillonet S (2007) Viral vectors for the expression of proteins in plants. Curr Opin Biotechnol. 18:134-41.
Gordon SP, Heisler MG, Reddy GV, Ohno C, Das P and Meyerowitz EM (2007) Pattern formation during de novo assembly of the Arabidopsis shoot meristem. Development (Cambridge, England) 134: 3539-3548.
Hagemann R (2004) The Sexual Inheritance of Plant Organelles, in Molecular Biology and Biotechnology of Plant Organelles pp 93-113.
Halioua E (2006) Status of biomanufacturing in the world. Eurobio conference 2006. www.eurobio2006.com/DocBD/press/pdf/41.pdf (accessed 3/3/09)
Hellwig S, Drossard J, Twyman RM and Fischer R (2004) Plant cell cultures for the production of recombinant proteins. Nature biotechnology 22: 1415-1422.
Huang CY, Ayliffe MA and Timmis JN (2003) Direct measurement of the transfer rate of chloroplast DNA into the nucleus. Nature 422: 72-76.
Huang J, Sutliff TD, Wu L, Nandi S, Benge K, Terashima M, Ralston AH, Drohan W, Huang N and Rodriguez RL (2001) Expression and purification of functional human alpha-1-Antitrypsin from cultured plant cells. Biotechnology progress 17:126-133.
Kanamoto H, Yamashita A, Asao H, Okumura S, Takase H, Hattori M, Yokota A and Tomizawa K (2006) Efficient and stable transformation of Lactuca sativa L. cv. Cisco (lettuce) plastids. Transgenic research 15: 205-217.
Kang TJ, Loc NH, Jang MO, Jang YS, Kim YS, Seo JE and Yang MS (2003) Expression of the B subunit of E. coli heat-labile enterotoxin in the chloroplasts of plants and its characterization. *Transgenic research* **12**: 683-691.
Khan MS and Maliga P (1999) Fluorescent antibiotic resistance marker for tracking plastid transformation in higher plants. Nature biotechnology 17: 910-915.
Kwon TH, Seo JE, Kim J, Lee JH, Jang YS and Yang MS (2003) Expression and secretion of the heterodimeric protein interleukin-12 in plant cell suspension culture. Biotechnology and bioengineering 81: 870-875.
Langbecker CL, Ye GN, Broyles DL, Duggan LL, Xu CW, Hajdukiewicz PT, Armstrong CL and Staub JM (2004) High-frequency transformation of undeveloped plastids in tobacco suspension cells. Plant physiology 135: 39-46.
Lee JH, Kim NS, Kwon TH, Jang YS and Yang MS (2002) Increased production of human granulocyte-macrophage colony stimulating factor (hGM-CSF) by the addition of stabilizing polymer in plant suspension cultures. Journal of biotechnology 96: 205-211.
Lelivelt CL, McCabe MS, Newell CA, Desnoo CB, van Dun KM, Birch-Machin I, Gray JC, Mills KH and Nugent JM (2005) Stable plastid transformation in lettuce (Lactuca sativa L.). Plant molecular biology 58: 763-774.
Ma JK, Drake PM and Christou P (2003) The production of recombinant pharmaceutical proteins in plants. Nature reviews 4: 794-805.
Maliga P (2004) Plastid transformation in higher plants. Annu Rev Plant Biol. 55:289-313.
Molina A, Hervas-Stubbs S, Daniell H, Mingo-Castel AM and Veramendi J (2004) High-yield expression of a viral peptide animal vaccine in transgenic tobacco chloroplasts. Plant biotechnology journal 2: 141-153.
Monier JM, Bernillon D, Kay E, Faugier A, Rybalka O, Dessaux Y, Simonet P and Vogel TM (2007) Detection of potential transgenic plant DNA recipients among soil bacteria. Environmental biosafety research 6: 71-83.
Murashige K and Skoog F (1962) A revised medium for rapid growth and bio assays with Tobacco tissue cultures. Physiologia Plantarum 15: 473:497.
Newell CA, Birch-Machin I, Hibberd JM and Gray JC (2003) Expression of green fluorescent protein from bacterial and plastid promoters in tobacco chloroplasts. Transgenic research 12: 631-634.
Ruf S, Hermann M, Berger IJ, Carrer H and Bock R (2001) Stable genetic transformation of tomato plastids and expression of a foreign protein in fruit. Nature biotechnology 19: 870-875.
Ruf S, Karcher D and Bock R (2007) Determining the transgene containment level provided by chloroplast transformation. Proceedings of the National Academy of Sciences of the United States of America 104: 6998-7002.
Saxby S (1999) Commercial production of monoclonal antibodies. In: Proceedings of the Production of Monoclonal antibodies Workshop (Eds. McArdle JE and Lund CJ). http://altweb.jhsph.edu/topics/mabs/ardf/saxby.htm (accessed 3/2/09)
Scholz O, Thiel A, Hillen W and Niederweis M (2000) Quantitative analysis of gene expression with an improved green fluorescent protein. p6. European journal of biochemistry / FEBS 267: 1565-1570.
Scott SE and Wilkinson MJ (1999) Low probability of chloroplast movement from oilseed rape (Brassica napus) into wild Brassica rapa. Nature biotechnology 17: 390-392.
Sheppard AE, Ayliffe MA, Blatch L, Day A, Delaney SK, Khairul-Fahmy N, Li Y, Madesis P, Pryor AJ and Timmis JN (2008) Transfer of plastid DNA to the nucleus is elevated during male gametogenesis in tobacco. Plant physiology 148: 328-336.
Sidorov VA, Kasten D, Pang SZ, Hajdukiewicz PT, Staub JM and Nehra NS (1999) Technical Advance: Stable chloroplast transformation in potato: use of green fluorescent protein as a plastid marker. Plant J 19: 209-216.
Silhavy D and Maliga P (1998) Plastid promoter utilization in a rice embryogenic cell culture. Current genetics 34: 67-70.
Svab Z, Hajdukiewicz P and Maliga P (1990) Stable transformation of plastids in higher plants. Proceedings of the National Academy of Sciences of the United States of America 87: 8526-8530.
Svab Z and Maliga P (2007) Exceptional transmission of plastids and mitochondria from the transplastomic pollen parent and its impact on transgene containment. Proceedings of the National Academy of Sciences of the United States of America 104: 7003-7008.
Terrier B, Courtois D, Henault N, Cuvier A, Bastin M, Aknin A, Dubreuil J and Petiard V (2007) Two new disposable bioreactors for plant cell culture: The wave and undertow bioreactor and the slug bubble bioreactor. Biotechnology and bioengineering 96: 914-923.
Tregoning JS, Nixon P, Kuroda H, Svab Z, Clare S, Bowe F, Fairweather N, Ytterberg J, van Wijk KJ, Dougan G and Maliga P (2003) Expression of tetanus toxin Fragment C in tobacco chloroplasts. Nucleic acids research 31: 1174-1179.
Twyman RM, Stoger E, Schillberg S, Christou P and Fischer R (2003) Molecular farming in plants: host systems and expression technology. Trends in biotechnology 21: 570-578.
Verma D and Daniell H (2007) Chloroplast vector systems for biotechnology applications. Plant Physiology 145: 1129-1143.
Wang T, Li Y, Shi Y, Reboud X, Darmency H and Gressel J (2004) Low frequency transmission of a plastid-encoded trait in Setaria italica. Theoretical and applied genetics 108: 315-320.
Wilke D and Katzek J (2003) Primary production of biopharmaceuticals in plants - An economically attractive choice? In: European Biopharmaceutical Review (Autumn). www.biomitteldeutschland.de/files/pdf/Biopharmaceuticalsinplants.pdf (accessed 3/2/09)
Zhang Q, Liu Y and Sodmergen (2003) Examination of the Cytoplasmic DNA in Male Reproductive Cells to Determine the Potential for Cytoplasmic Inheritance in 295 Angiosperm Species, in Plant Cell Physiology 44: 941-951.

## Claims

1. A method for producing leafy biomass from undifferentiated plant cells, the method comprising providing undifferentiated plant cells, contacting them with an agent that promotes differentiation of the cells into leafy tissue and growing the cells in suspension in a temporary liquid immersion culture system; and wherein the agent is present in the culture medium of the temporary liquid immersion culture system; and wherein the agent is a cytokinin; and wherein the agent is added in the culture medium at a concentration of from 0.01 to 100µM, and wherein the immersion time varies from 1 to 30 minutes every 2 to 24 hours, and wherein the volume of liquid in the temporary liquid immersion culture is from 1 to 10,000 litres.

2. A method according to Claim 1, wherein the plant cells are cells from (i) a monocotyledon selected from corn, rye, oat, millet, sugar cane, sorghum, maize, wheat, rice, Miscanthus sp, Panicum sp, or palm tree or (ii) a dicotyledon selected from tobacco, tomato, potato, bean, soybean, carrot, cassava, *Arabidopsis, Atropa sp, Hyoscyamus sp, Datura sp, Papaver sp, Scopolia sp, Digitalis sp, Macuna sp, Taxus sp, Camptotheca sp, Catharanthus sp. Artemisia sp, Jatropha sp,* Willow or Poplar; or (iii) *Cephalotaxus sp.*

3. A method according to either Claim 1 or 2 wherein the cytokinin is any of adenine, kinetin, zeatin, 6-benzylaminopurine, diphenylurea or thidiazuron (TDZ).

4. A method according to any of Claims 1 to 3 wherein the agent is used in combination with an auxin.

5. A method according to any of the preceding claims wherein the plant cells are not genetically engineered.

6. A method according to any of Claims 1 to 5 wherein the plant cells are genetically engineered.

7. A method of producing a polypeptide in plant cells *in vitro* comprising:
providing undifferentiated plant cells containing chloroplasts that carry a transgenic nucleic acid molecule encoding the polypeptide, wherein the plant cells display homoplastomy; and
propagating the cells according to the method of Claim 1 to produce leafy biomass containing the polypeptide.

8. A method according to Claim 7, wherein the step of providing the undifferentiated cells comprises:
introducing the transgenic nucleic acid molecule into a chloroplast of a plant cell;
inducing the plant cell containing the transgenic nucleic acid molecule to form a callus of undifferentiated cells; and
propagating the callus under conditions effective to achieve homoplastomy.

9. A method according to Claim 7 or 8 wherein homoplastomy is achieved using antibiotic selection.

10. A method according to any of Claims 7 to 9 wherein the amount of light available and/or the amount of sucrose available is controlled to optimise production of the polypeptide.

11. A method according to any of Claims 7 to 10, further comprising obtaining the polypeptide from the leafy biomass.

12. A method according to any of Claims 7 to 11, wherein the polypeptide is any one of a therapeutic polypeptide, an enzyme, a growth factor, an immunoglobulin, a hormone, a structural protein, a protein involved in stress responses of a plant, a biopharmaceutical or a vaccine antigen.

13. A method for obtaining a component present in leafy biomass, the method comprising producing leafy biomass according to any of Claims 1 to 6, and obtaining the component from the leafy biomass.

14. A method according to claim 13 wherein the component is obtained by its secretion by the leafy biomass or by extraction from the leafy biomass.

15. A method according to Claim 13 or 14 wherein the component is a medicinal product, a recombinantly expressed polypeptide, a carbohydrate, a lipid, an oil, a volatile aromatic compound, an anti-oxidants, a pigment, a flavour or flavour precursor; and wherein the component may be either endogenous or exogenous.

16. A method according to Claims 13 to 15 wherein the component is processed into a further product.

17. A method of capturing carbon dioxide, the method comprising carrying out the method of Claims 1 to 6.

## Patentansprüche

1. Verfahren zum Herstellen von Blattbiomasse aus undifferenzierten Pflanzenzellen, wobei das Verfahren das Bereitstellen undifferenzierter Pflanzenzellen, das Inkontaktbringen derselben mit einem Mittel, das die Differenzierung der Zellen zu Blattgewebe fördert und das Züchten der Zellen in Suspension in einem temporären flüssigen Immersionskultursystem umfasst; und wobei das Mittel in dem Kulturmedium des temporären flüssigen Immersionskultursystems vorhanden ist; und wobei das Mittel ein Cytokinin ist; und wobei das Mittel in dem Kulturmedium in einer Konzentration von 0,01 bis 100 µM zugegeben wird, und wobei die Immersionsdauer von 1 bis 30 Minuten alle 2 bis 24 Stunden variiert, und wobei das Volumen der Flüssigkeit in dem temporären flüssigen Immersionskultursystem von 1 bis 10000 Litern reicht.

2. Verfahren nach Anspruch 1, wobei es sich bei den Pflanzenzellen um Zellen aus (i) einer Monocotylen, ausgewählt aus Mais, Roggen, Hafer, Hirse, Zuckerrohr, Sorghum, Mais, Weizen, Reis, Miscanthus sp., Panicum sp. oder Palme, oder (ii) einer Dicotylen, ausgewählt aus Tabak, Tomate, Kartoffel, Bohne, Sojabohne, Karotte, Maniok, *Arabidopsis, Atropa sp. , Hyoscyamus sp. , Datura* sp., *Papaver sp., Scopolia sp., Digitalis sp., Macuna sp., Taxus sp., Camptotheca sp., Catharanthus sp., Artemisia sp., Jatropha sp.,* Weide oder Pappel; oder (iii) Cephalotaxus sp. handelt.

3. Verfahren nach Anspruch 1 oder 2, wobei das Cytokinin eine beliebige Substanz von Adenin, Kinetin, Zeatin, 6-Benzylaminopurin, Diphenylharnstoff, Thidiazuron (TDZ) ist.

4. Verfahren nach einem der Ansprüche 1 bis 3, wobei das Mittel in Kombination mit einem Auxin verwendet wird.

5. Verfahren nach einem der vorhergehenden Ansprüche, wobei die Pflanzenzellen nicht gentechnisch verändert sind.

6. Verfahren nach einem der Ansprüche 1 bis 5, wobei die Pflanzenzellen gentechnisch verändert sind.

7. Verfahren zur Herstellung eines Polypeptids in Pflanzenzellen *in vitro,* umfassend:
Bereitstellen undifferenzierter Pflanzenzellen, die Chloroplasten enthalten, die ein transgenes Nukleinsäuremolekül tragen, das das Polypeptid kodiert, wobei die Pflanzenzellen eine Homoplastomie zeigen; und
Vermehren der Zellen gemäß dem Verfahren von Anspruch 1, um eine Blattbiomasse zu erzeugen, die das Polypeptid enthält.

8. Verfahren nach Anspruch 7, wobei der Schritt des Bereitstellens der undifferenzierten Zellen Folgendes umfasst:
Einführen des transgenen Nukleinsäuremoleküls in einen Chloroplasten einer Pflanzenzelle;
Induzieren der Pflanzenzelle, die das transgene Nukleinsäuremolekül enthält, um einen Kallus von undifferenzierten Zellen zu erhalten; und
Vermehren des Kallus unter solchen Bedingungen, dass eine Homoplastomie wirksam erreicht wird.

9. Verfahren nach Anspruch 7 oder 8, wobei die Homoplastomie unter Verwendung von Antibiotikaselektion erreicht wird.

10. Verfahren nach einem der Ansprüche 7 bis 9, wobei die zur Verfügung stehende Lichtmenge und/oder die zur Verfügung stehende Saccharosemenge derart gesteuert wird/werden, dass die Produktion des Polypeptids optimiert wird.

11. Verfahren nach einem der Ansprüche 7 bis 10, ferner umfassend das Erhalten des Polypeptids aus der Blattbiomasse.

12. Verfahren nach einem der Ansprüche 7 bis 11, wobei das Polypeptid eine beliebige Substanz von einem therapeutischen Polypeptid, einem Enzym, einem Wachstumsfaktor, einem Immunglobulin, einem Hormon, einem Strukturprotein, einem Protein, das an Stressreaktionen einer Pflanze beteiligt ist, einem Biopharmazeutikum oder einem Impfstoffantigen ist.

13. Verfahren zum Gewinnen einer in Blattbiomasse vorhandenen Komponente, wobei das Verfahren das Herstellen von Blattbiomasse nach einem der Ansprüche 1 bis 6 und das Gewinnen der Komponente aus der Blattbiomasse umfasst.

14. Verfahren nach Anspruch 13, wobei die Komponente durch ihre Sekretion durch die Blattbiomasse oder durch Extraktion aus der Blattbiomasse gewonnen wird.

15. Verfahren nach Anspruch 13 oder 14, wobei die Komponente ein medizinisches Produkt, ein rekombinant exprimiertes Polypeptid, ein Kohlenhydrat, ein Lipid, ein Öl, eine flüchtige aromatische Verbindung, ein Antioxidans, ein Pigment, ein Geschmacksstoff oder eine Geschmacksstoffvorstufe ist; und wobei die Komponente entweder endogen oder exogen sein kann.

16. Verfahren nach den Ansprüchen 13 bis 15, wobei die Komponente zu einem weiteren Produkt verarbeitet wird.

17. Verfahren zum Einfangen von Kohlendioxid, wobei das Verfahren das Durchführen des Verfahrens nach den Ansprüchen 1 bis 6 umfasst.

## Revendications

1. Méthode de production de biomasse feuillée à partir de cellules végétales non différenciées, la méthode comprenant la fourniture de cellules végétales non différenciées, leur mise en contact avec un agent qui favorise la différentiation des cellules en tissu feuillé et la culture des cellules en suspension dans un système de culture par immersion liquide temporaire ; et où l'agent est présent dans le milieu de culture du système de culture par immersion liquide temporaire ; et où l'agent est une cytokinine ; et où l'agent est ajouté au milieu de culture selon une concentration allant de 0,01 à 100 µM, et où le temps d'immersion varie de 1 à 30 minutes toutes les 2 à 24 heures, et où le volume de liquide dans la culture par immersion liquide temporaire va de 1 à 10 000 litres.

2. Méthode selon la revendication 1, dans laquelle les cellules végétales sont des cellules issues (i) d'une monocotylédone choisie parmi le maïs, le seigle, l'avoine, le millet, la canne à sucre, le sorgho, le maïs, le blé, le riz, *Miscanthus* sp., *Panicum* sp., ou le palmier ou (ii) d'une dicotylédone choisie parmi le tabac, la tomate, la pomme de terre, le haricot, le soja, la carotte, le manioc, *Arabidopsis, Atropa* sp., *Hyoscyamus* sp., *Datura* sp., *Papaver* sp., *Scopolia* sp., *Digitalis* sp., *Macuna* sp., *Taxus* sp., *Camptotheca* sp., *Catharanthus* sp., *Artemisia* sp., *Jatropha* sp., le bouleau ou le peuplier ; ou (iii) de *Cephalotaxus* sp.

3. Méthode selon la revendication 1 ou 2, dans laquelle la cytokinine est l'une quelconque parmi l'adénine, la kinétine, la zéatine, la 6-benzylaminopurine, la diphénylurée ou le thidiazuron (TDZ).

4. Méthode selon l'une quelconque des revendications 1 à 3, dans laquelle l'agent est utilisé en combinaison avec une auxine.

5. Méthode selon l'une quelconque des revendications précédentes, dans laquelle les cellules végétales ne sont pas modifiées génétiquement.

6. Méthode selon l'une quelconque des revendications 1 à 5, dans laquelle les cellules végétales sont modifiées génétiquement.

7. Méthode de production d'un polypeptide dans des cellules végétales *in vitro,* comprenant :
la fourniture de cellules végétales non différenciées contenant des chloroplastes portant une molécule d'acide nucléique transgénique codant pour le polypeptide, où les cellules végétales présentent une homoplastomie ; et
la propagation des cellules selon la méthode de la revendication 1, afin de produire une biomasse feuillée contenant le polypeptide.

8. Méthode selon la revendication 7, dans laquelle l'étape consistant à fournir les cellules non différenciées comprend :
l'introduction de la molécule d'acide nucléique transgénique dans un chloroplaste d'une cellule végétale ;
l'induction de la cellule végétale contenant la molécule d'acide nucléique transgénique afin de former un calle de cellules non différentiées ; et
la propagation du calle dans des conditions efficaces pour obtenir une homoplastomie.

9. Méthode selon la revendication 7 ou 8, dans laquelle l'homoplastomie est obtenue en utilisant une sélection par antibiotiques.

10. Méthode selon l'une quelconque des revendications 7 à 9, dans laquelle la quantité de lumière disponible et/ou la quantité de saccharose disponible est contrôlée afin d'optimiser la production du polypeptide.

11. Méthode selon l'une quelconque des revendications 7 à 10, comprenant en outre l'obtention du polypeptide à partir de la biomasse feuillée.

12. Méthode selon l'une quelconque des revendications 7 à 11, dans laquelle le polypeptide est l'un quelconque parmi un polypeptide thérapeutique, une enzyme, un facteur de croissance, une immunoglobuline, une hormone, une protéine structurelle, une protéine impliquée dans les réponses au stress chez une plante, une substance biopharmaceutique ou un antigène de vaccin.

13. Méthode d'obtention d'un composant présent dans une biomasse feuillée, la méthode comprenant la production de biomasse feuillée selon l'une quelconque des revendications 1 à 6, et l'obtention du composant à partir de la biomasse feuillée.

14. Méthode selon la revendication 13, dans laquelle le composant est obtenu par sa sécrétion par la biomasse feuillée ou par extraction à partir de la biomasse feuillée.

15. Méthode selon la revendication 13 ou 14, dans laquelle le composant est un produit médicinal, un polypeptide exprimé de manière recombinée, un glucide, un lipide, une huile, un composé aromatique volatil, un antioxydant, un pigment, un arôme ou un précurseur d'arôme ; et où le composant peut être endogène ou bien exogène.

16. Méthode selon les revendications 13 à 15, dans laquelle le composant est traité en un produit supplémentaire.

17. Méthode de piégeage de dioxyde de carbone, la méthode comprenant la mise en oeuvre de la méthode selon les revendications 1 à 6.
